# EUROPEAN PATENT SPECIFICATION

(11) **EP 0 862 742 B1**
(45) Date of publication and mention of the grant of the patent: **11.06.2003**
(21) Application number: 96944747.3
(22) Date of filing: 22.11.1996
(51) Int. Cl.: G01N 33/86, C12Q 1/56

(54) **ASSAY FOR BLOOD CLOTTING FACTORS VIII AND IX**
VERFAHREN ZUR BESTIMMUNG VON BLUTGERINNUNGSFAKTOREN VIII UND IX
ESSAI DE DOSAGE POUR LES FACTEURS DE COAGULATION SANGUINE VIII ET IX

(30) Priority: 22.11.1995 US 7474 P
(43) Date of publication of application: 09.09.1998
(73) Proprietor: Oklahoma Medical Research Foundation, Oklahoma City Oklahoma 73104 (US)
(72) Inventor: MORRISSEY, James, H., Oklahoma City, OK 73118 (US)
(74) Representative: Warcoin, Jacques
(86) International application number: US9619275
(87) International publication number: WO97019357

(56) References cited:
- EP-A- 0 566 333
- WO-A-93/07492
- WO-A-93/23074
- WO-A-94/07515
- WO-A-96/01653
- US-A- 5 346 991
- THE JOURNAL OF BIOLOGICAL CHEMISTRY, vol. 266, no. 32, 1991, pages 22063-22066, XP002028728 SOUMITRA ROY, ET AL.: "Lysine residues 165 and 166 are essential for the cofactor function of Tissue Factor" cited in the application
- THE JOURNAL OF BIOLOGICAL CHEMISTRY, vol. 267, no. 31, 1992, pages 22206-22210, XP002028729 WOLFRAM RUF, ET AL.: "Tissue factor residues 157-167 are required for efficient proteolytic activation of factor X and Factor VII." cited in the application
- THE JOURNAL OF BIOLOGICAL CHEMISTRY, vol. 266, no. 32, 1991, pages 21911-21916, XP002028730 LISA R. PABORSKY, ET AL.: "Lipid association, but not the transmembrane domain, is required for tissue factor activity" cited in the application
- THROMB. HAEMOSTASIS (1991), 66(4), 430-4 CODEN: THHADQ;ISSN: 0340-6245, 1991, XP002028731 KESSELS, H. ET AL: "A method for measuring activated factor VIII in plasma"

### SEQUENCE LISTING

(1) GENERAL INFORMATION:
   (i) APPLICANT:
      (A) NAME: Oklahoma Medical Research Foundation
      (B) STREET: 825 NE 13th Street
      (C) CITY: Oklahoma City
      (D) STATE: Oklahoma
      (E) COUNTRY: US
      (F) POSTAL CODE (ZIP): 73104
      (G) TELEPHONE: (405) 271-6673
      (H) TELEFAX: (405) 271-3980
   (ii) TITLE OF INVENTION: ASSAY FOR BLOOD CLOTTING FACTORS VIII AND IX
   (iii) NUMBER OF SEQUENCES: 48
   (iv) CORRESPONDENCE ADDRESS:
      (A) ADDRESSEE: SIDLEY & AUSTIN
      (B) STREET: 1201 Elm Street, Suite 4500
      (C) CITY: Dallas
      (D) STATE: Texas
      (E) COUNTRY: United States of America
      (F) ZIP: 75270-2197
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentin Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Hansen, Eugenia S.
      (B) REGISTRATION NUMBER: 31,966
      (C) REFERENCE/DOCKET NUMBER: 11146/08501
   (ix) TELECOMMUNICATION INFORMATION:
      (A) TELEPHONE: 214-981-3300
      (B) TELEFAX: 214-981-3400
(2) INFORMATION FOR SEQ ID NO:1:
   (i) SEQUENCE CHARACTERISTICS:
      (A) LENGTH: 2141 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Homo sapiens
   (ix) FEATURE:
      (A) NAME/KEY: CDS
      (B) LOCATION: 112..999
   (ix) FEATURE:
      (A) NAME/KEY: mat_peptide
      (B) LOCATION: 208..996
      (D) OTHER INFORMATION: /note= "TISSUE FACTOR VERSION 1"
   (ix) FEATURE:
      (A) NAME/KEY: mat_peptide
      (B) LOCATION: 214..996
      (D) OTHER INFORMATION: /note= "TISSUE FACTOR VERSION 2"
   (xi) SEQUENCE DESCRIPTION: SEQ ID NO:1:
   CGGGCGAACC CCCTCGCACT CCCTCTGGCC GGCCCAGGGC GCCTTCAGCC CAACCTCCCC        60
   AGCCCCACGG GCGCCACGGA ACCCGCTCGA TCTCGCCGCC AACTGGTAGA C ATG GAG        117
                                                            Met Glu
                                                            -32
   ACC CCT GCC TGG CCC CGG GTC CCG CGC CCC GAG ACC GCC GTC GCT CGG         165
   Thr Pro Ala Trp Pro Arg Val Pro Arg Pro Glu Thr Ala Val Ala Arg
   -30                 -25                 -20                 -15
   ACG CTC CTG CTC GGC TGG GTC TTC GCC CAG GTG GCC GGC GCT TCA GGC         213
   Thr Leu Leu Leu Gly Trp Val Phe Ala Gln Val Ala Gly Ala Ser Gly
                   -10                  -5                   1
   ACT ACA AAT ACT GTG GCA GCA TAT AAT TTA ACT TGG AAA TCA ACT AAT         261
   Thr Thr Asn Thr Val Ala Ala Tyr Asn Leu Thr Trp Lys Ser Thr Asn
               5                  10                  15
   TTC AAG ACA ATT TTG GAG TGG GAA CCC AAA CCC GTC AAT CAA GTC TAC         309
   Phe Lys Thr Ile Leu Glu Trp Glu Pro Lys Pro Val Asn Gln Val Tyr
       20                  25                  30
   ACT GTT CAA ATA AGC ACT AAG TCA GGA GAT TGG AAA AGC AAA TGC TTT      357
   Thr Val Gln Ile Ser Thr Lys Ser Gly Asp Trp Lys Ser Lys Cys Phe
    35             40                  45                  50
   TAC ACA ACA GAC ACA GAG TGT GAC CTC ACC GAC GAG ATT GTG AAG GAT      405
   Tyr Thr Thr Asp Thr Glu Cys Asp Leu Thr Asp Glu Ile Val Lys Asp
                    55                  60                  65
   GTG AAG CAG ACG TAC TTG GCA CGG GTC TTC TCC TAC CCG GCA GGG AAT      453
   Val Lys Gln Thr Tyr Leu Ala Arg Val Phe Ser Tyr Pro Ala Gly Asn
                70                  75                  80
   GTG GAG AGC ACC GGT TCT GCT GGG GAG CCT CTG TAT GAG AAC TCC CCA      501
   Val Glu Ser Thr Gly Ser Ala Gly Glu Pro Leu Tyr Glu Asn Ser Pro
            85                  90                  95
   GAG TTC ACA CCT TAC CTG GAG ACA AAC CTC GGA CAG CCA ACA ATT CAG      549
   Glu Phe Thr Pro Tyr Leu Glu Thr Asn Leu Gly Gln Pro Thr Ile Gln
       100                 105                 110
   AGT TTT GAA CAG GTG GGA ACA AAA GTG AAT GTG ACC GTA GAA GAT GAA      597
   Ser Phe Glu Gln Val Gly Thr Lys Val Asn Val Thr Val Glu Asp Glu
   115                 120                 125                 130
   CGG ACT TTA GTC AGA AGG AAC AAC ACT TTC CTA AGC CTC CGG GAT GTT      645
   Arg Thr Leu Val Arg Arg Asn Asn Thr Phe Leu Ser Leu Arg Asp Val
                   135                 140                 145
   TTT GGC AAG GAC TTA ATT TAT ACA CTT TAT TAT TGG AAA TCT TCA AGT      693
   Phe Gly Lys Asp Leu Ile Tyr Thr Leu Tyr Tyr Trp Lys Ser Ser Ser
               150                 155                 160
   TCA GGA AAG AAA ACA GCC AAA ACA AAC ACT AAT GAG TTT TTG ATT GAT      741
   Ser Gly Lys Lys Thr Ala Lys Thr Asn Thr Asn Glu Phe Leu Ile Asp
           165                 170                 175
   GTG GAT AAA GGA GAA AAC TAC TGT TTC AGT GTT CAA GCA GTG ATT CCC      789
   Val Asp Lys Gly Glu Asn Tyr Cys Phe Ser Val Gln Ala Val Ile Pro
       180                 185                 190
   TCC CGA ACA GTT AAC CGG AAG AGT ACA GAC AGC CCG GTA GAG TGT ATG      837
   Ser Arg Thr Val Asn Arg Lys Ser Thr Asp Ser Pro Val Glu Cys Met
   195                 200                 205                 210
   GGC CAG GAG AAA GGG GAA TTC AGA GAA ATA TTC TAC ATC ATT GGA GCT      885
   Gly Gln Glu Lys Gly Glu Phe Arg Glu Ile Phe Tyr Ile Ile Gly Ala
                   215                 220                 225
   GTG GTA TTT GTG GTC ATC ATC CTT GTC ATC ATC CTG GCT ATA TCT CTA      933
   Val Val Phe Val Val Ile Ile Leu Val Ile Ile Leu Ala Ile Ser Leu
               230                 235                 240
   CAC AAG TGT AGA AAG GCA GGA GTG GGG CAG AGC TGG AAG GAG AAC TCC        981
   His Lys Cys Arg Lys Ala Gly Val Gly Gln Ser Trp Lys Glu Asn Ser
           245                     250                     255
   CCA CTG AAT GTT TCA TAA AGGAAGCACT GTTGGAGCTA CTGCAAATGC              1029
   Pro Leu Asn Val Ser  *
       260
   TATATTGCAC TGTGACCGAG AACTTTTAAG AGGATAGAAT ACATGGAAAC GCAAATGAGT    1089
   ATTTCGGAGC ATGAAGACCC TGGAGTTCAA AAAACTCTTG ATATGACCTG TTATTACCAT    1149
   TAGCATTCTG GTTTTGACAT CAGCATTAGT CACTTTGAAA TGTAACGAAT GGTACTACAA    1209
   CCAATTCCAA GTTTTAATTT TTAACACCAT GGCACCTTTT GCACATAACA TGCTTTAGAT    1269
   TATATATTCC GCACTTAAGG ATTAACCAGG TCGTCCAAGC AAAAACAAAT GGGAAAATGT    1329
   CTTAAAAAAT CCTGGGTGGA CTTTTGAAAA GCTTTTTTTT TTTTTTTTTT TTGAGACGGA    1389
   GTCTTGCTCT GTTGCCCAGG CTGGAGTGCA GTAGCACGAT CTCGGCTCAC TTGCACCCTC    1449
   CGTCTCTCGG GTTCAAGCAA TTGTCTGCCT CAGCCTCCCG AGTAGCTGGG ATTACAGGTG    1509
   CGCACTACCA CGCCAAGCTA ATTTTTGTAT TTTTTAGTAG AGATGGGGTT TCACCATCTT    1569
   GGCCAGGCTG GTCTTGAATT CCTGACCTCA GTGATCCACC CACCTTGGCC TCCCAAAGAT    1629
   GCTAGTATTA TGGGCGTGAA CCACCATGCC CAGCCGAAAA GCTTTTGAGG GGCTGACTTC    1689
   AATCCATGTA GGAAAGTAAA ATGGAAGGAA ATTGGGTGCA TTTCTAGGAC TTTTCTAACA    1749
   TATGTCTATA ATATAGTGTT TAGGTTCTTT TTTTTTTCAG GAATACATTT GGAAATTCAA    1809
   AACAATTGGG CAAACTTTGT ATTAATGTGT TAAGTGCAGG AGACATTGGT ATTCTGGGCA    1869
   GCTTCCTAAT ATGCTTTACA ATCTGCACTT TAACTGACTT AAGTGGCATT AAACATTTGA    1929
   GAGCTAACTA TATTTTTATA AGACTACTAT ACAAACTACA GAGTTTATGA TTTAAGGTAC    1989
   TTAAAGCTTC TATGGTTGAC ATTGTATATA TAATTTTTTA AAAAGGTTTT TCTATATGGG    2049
   GATTTTCTAT TTATGTAGGT AATATTGTTC TATTTGTATA TATTGAGATA ATTTATTTAA    2109
   TATACTTTAA ATAAAGGTGA CTGGGAATTG TT                                   2141
(2) INFORMATION FOR SEQ ID NO:2:
   (i) SEQUENCE CHARACTERISTICS:
      (A) LENGTH: 296 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Glu Thr Pro Ala Trp Pro Arg Val Pro Arg Pro Glu Thr Ala Val
   -32     -30                 -25                 -20
   Ala Arg Thr Leu Leu Leu Gly Trp Val Phe Ala Gln Val Ala Gly Ala
       -15                 -10                 -5
   Ser Gly Thr Thr Asn Thr Val Ala Ala Tyr Asn Leu Thr Trp Lys Ser
    1               5                  10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser *
   260
(2) INFORMATION FOR SEQ ID NO:3:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:3:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
   50 55 60
   Lys Asp Val Lys Gln Thr Tyr Leu Ala Arg Val Phe Ser Tyr Pro Ala
   65 70 75 80
   Gly Asn Val Glu Ser Thr Gly Ser Ala Gly Glu Pro Leu Tyr Glu Asn
   85 90 95
   Ser Pro Glu Phe Thr Pro Tyr Leu Glu Thr Asn Leu Gly Gln Pro Thr
   100 105 110
   Ile Gln Ser Phe Glu Gln Val Gly Thr Lys Val Asn Val Thr Val Glu
   115 120 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Gln Lys Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser
   260
(2) INFORMATION FOR SEQ ID NO:4:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:4:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
   50 55 60
   Lys Asp Val Lys Gln Thr Tyr Leu Ala Arg Val Phe Ser Tyr Pro Ala
   65 70 75 80
   Gly Asn Val Glu Ser Thr Gly Ser Ala Gly Glu Pro Leu Tyr Glu Asn
   85 90 95
   Ser Pro Glu Phe Thr Pro Tyr Leu Glu Thr Asn Leu Gly Gln Pro Thr
   100 105 110
   Ile Gln Ser Phe Glu Gln Val Gly Thr Lys Val Asn Val Thr Val Glu
   115 120 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Ala Lys Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser
   260
(2) INFORMATION FOR SEQ ID NO:5:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:5:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Glu Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                 240
   Ser Leu His Lys Cys Arg Lys Ala Gly Val Gly Gln Ser Trp Lys Glu
                   245                 250                 255
   Asn Ser Pro Leu Asn Val Ser
               260
(2) INFORMATION FOR SEQ ID NO:6:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:6:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Gln Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser
   260
(2) INFORMATION FOR SEQ ID NO:7:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:7:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
   50 55 60
   Lys Asp Val Lys Gln Thr Tyr Leu Ala Arg Val Phe Ser Tyr Pro Ala
   65 70 75 80
   Gly Asn Val Glu Ser Thr Gly Ser Ala Gly Glu Pro Leu Tyr Glu Asn
   85 90 95
   Ser Pro Glu Phe Thr Pro Tyr Leu Glu Thr Asn Leu Gly Gln Pro Thr
   100 105 110
   Ile Gln Ser Phe Glu Gln Val Gly Thr Lys Val Asn Val Thr Val Glu
   115 120 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Lys Ala Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser
   260
(2) INFORMATION FOR SEQ ID NO:8:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:8:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Glu Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                 240
   Ser Leu His Lys Cys Arg Lys Ala Gly Val Gly Gln Ser Trp Lys Glu
                   245                 250                 255
   Asn Ser Pro Leu Asn Val Ser
               260
(2) INFORMATION FOR SEQ ID NO:9:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:9:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Gln Gln Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                     240
   Ser Leu His Lys Cys Arg Lys Ala Gly Val Gly Gln Ser Trp Lys Glu
                   245                 250                 255
   Asn Ser Pro Leu Asn Val Ser
               260
(2) INFORMATION FOR SEQ ID NO:10:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:10:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
   100 105 110
   Ile Gln Ser Phe Glu Gln Val Gly Thr Lys Val Asn Val Thr Val Glu
   115 120 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Gln Ala Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser
   260
(2) INFORMATION FOR SEQ ID NO:11:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:11:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Ala Gln Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                 240
   Ser Leu His Lys Cys Arg Lys Ala Gly Val Gly Gln Ser Trp Lys Glu
                   245                 250                 255
   Asn Ser Pro Leu Asn Val Ser
               260
(2) INFORMATION FOR SEQ ID NO:12:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:12:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Gln Glu Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                     240
   Ser Leu His Lys Cys Arg Lys Ala Gly Val Gly Gln Ser Trp Lys Glu
                   245                 250                 255
   Asn Ser Pro Leu Asn Val Ser
               260
(2) INFORMATION FOR SEQ ID NO:13:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:13:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                      80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
   115 120 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Glu Gln Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser
   260
(2) INFORMATION FOR SEQ ID NO:14:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:14:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
   50 55 60
   Lys Asp Val Lys Gln Thr Tyr Leu Ala Arg Val Phe Ser Tyr Pro Ala
   65 70 75 80
   Gly Asn Val Glu Ser Thr Gly Ser Ala Gly Glu Pro Leu Tyr Glu Asn
   85 90 95
   Ser Pro Glu Phe Thr Pro Tyr Leu Glu Thr Asn Leu Gly Gln Pro Thr
   100 105 110
   Ile Gln Ser Phe Glu Gln Val Gly Thr Lys Val Asn Val Thr Val Glu
   115 120 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Ala Ala Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser
   260
(2) INFORMATION FOR SEQ ID NO:15:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:15:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
   50 55 60
   Lys Asp Val Lys Gln Thr Tyr Leu Ala Arg Val Phe Ser Tyr Pro Ala
   65 70 75 80
   Gly Asn Val Glu Ser Thr Gly Ser Ala Gly Glu Pro Leu Tyr Glu Asn
   85 90 95
   Ser Pro Glu Phe Thr Pro Tyr Leu Glu Thr Asn Leu Gly Gln Pro Thr
   100 105 110
   Ile Gln Ser Phe Glu Gln Val Gly Thr Lys Val Asn Val Thr Val Glu
   115 120 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Ala Glu Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser
   260
(2) INFORMATION FOR SEQ ID NO:16:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:16:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
   50 55 60
   Lys Asp Val Lys Gln Thr Tyr Leu Ala Arg Val Phe Ser Tyr Pro Ala
   65 70 75 80
   Gly Asn Val Glu Ser Thr Gly Ser Ala Gly Glu Pro Leu Tyr Glu Asn
   85 90 95
   Ser Pro Glu Phe Thr Pro Tyr Leu Glu Thr Asn Leu Gly Gln Pro Thr
   100 105 110
   Ile Gln Ser Phe Glu Gln Val Gly Thr Lys Val Asn Val Thr Val Glu
   115 120 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Glu Ala Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser
   260
(2) INFORMATION FOR SEQ ID NO:17:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:17:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
   50 55 60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Glu Glu Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                 240
   Ser Leu His Lys Cys Arg Lys Ala Gly Val Gly Gln Ser Trp Lys Glu
                   245                 250                 255
   Asn Ser Pro Leu Asn Val Ser
               260
(2) INFORMATION FOR SEQ ID NO:18:
   (i) SEQUENCE CHARACTERISTICS:
      (A) LENGTH: 795 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Homo sapiens
   (ix) FEATURE:
      (A) NAME/KEY: CDS
      (B) LOCATION: 34..789
   (ix) FEATURE:
      (A) NAME/KEY: mat_peptide
      (B) LOCATION: 130..789
      (D) OTHER INFORMATION: /note= "TRUNCATED TISSUE FACTOR"
   (ix) FEATURE:
      (A) NAME/KEY: sig_peptide
      (B) LOCATION: 1..33
   (xi) SEQUENCE DESCRIPTION: SEQ ID NO:18:
   CGTTCCGCTC GATCTCGCCG CCAACTGGTA GAC ATG GAG ACC CCT GCC TGG CCC        54
                                        Met Glu Thr Pro Ala Trp Pro
                                        -32     -30
   CGG GTC CCG CGC CCC GAG ACC GCC GTC GCT CGG ACG CTC CTG CTC GGC        102
   Arg Val Pro Arg Pro Glu Thr Ala Val Ala Arg Thr Leu Leu Leu Gly
   -25                 -20                 -15                 -10
   TGG GTC TTC GCC CAG GTG GCC GGC GCT TCA GGC ACT ACA AAT ACT GTG        150
   Trp Val Phe Ala Gln Val Ala Gly Ala Ser Gly Thr Thr Asn Thr Val
                   -5                   1               5
   GCA GCA TAT AAT TTA ACT TGG AAA TCA ACT AAT TTC AAG ACA ATT TTG        198
   Ala Ala Tyr Asn Leu Thr Trp Lys Ser Thr Asn Phe Lys Thr Ile Leu
           10                  15                  20
   GAG TGG GAA CCC AAA CCC GTC AAT CAA GTC TAC ACT GTT CAA ATA AGC        246
   Glu Trp Glu Pro Lys Pro Val Asn Gln Val Tyr Thr Val Gln Ile Ser
       25                  30                  35
   ACT AAG TCA GGA GAT TGG AAA AGC AAA TGC TTT TAC ACA ACA GAC ACA        294
   Thr Lys Ser Gly Asp Trp Lys Ser Lys Cys Phe Tyr Thr Thr Asp Thr
   40                  45                  50                  55
   GAG TGT GAC CTC ACC GAC GAG ATT GTG AAG GAT GTG AAG CAG ACG TAC        342
   Glu Cys Asp Leu Thr Asp Glu Ile Val Lys Asp Val Lys Gln Thr Tyr
                   60                  65                  70
   TTG GCA CGG GTC TTC TCC TAC CCG GCA GGG AAT GTG GAG AGC ACC GGT        390
   Leu Ala Arg Val Phe Ser Tyr Pro Ala Gly Asn Val Glu Ser Thr Gly
               75                  80                  85
   TCT GCT GGG GAG CCT CTG TAT GAG AAC TCC CCA GAG TTC ACA CCT TAC        438
   Ser Ala Gly Glu Pro Leu Tyr Glu Asn Ser Pro Glu Phe Thr Pro Tyr
            90                  95                 100
   CTG GAG ACA AAC CTC GGA CAG CCA ACA ATT CAG AGT TTT GAA CAG GTG        486
   Leu Glu Thr Asn Leu Gly Gln Pro Thr Ile Gln Ser Phe Glu Gln Val
       105                 110                 115
   GGA ACA AAA GTG AAT GTG ACC GTA GAA GAT GAA CGG ACT TTA GTC AGA        534
   Gly Thr Lys Val Asn Val Thr Val Glu Asp Glu Arg Thr Leu Val Arg
   120                 125                 130                 135
   AGG AAC AAC ACT TTC CTA AGC CTC CGG GAT GTT TTT GGC AAG GAC TTA        582
   Arg Asn Asn Thr Phe Leu Ser Leu Arg Asp Val Phe Gly Lys Asp Leu
                   140                 145                 150
   ATT TAT ACA CTT TAT TAT TGG AAA TCT TCA AGT TCA GGA AAG AAA ACA        630
   Ile Tyr Thr Leu Tyr Tyr Trp Lys Ser Ser Ser Ser Gly Lys Lys Thr
               155                 160                 165
   GCC AAA ACA AAC ACT AAT GAG TTT TTG ATT GAT GTG GAT AAA GGA GAA        678
   Ala Lys Thr Asn Thr Asn Glu Phe Leu Ile Asp Val Asp Lys Gly Glu
           170                 175                 180
   AAC TAC TGT TTC AGT GTT CAA GCA GTG ATT CCC TCC CGA ACA GTT AAC        726
   Asn Tyr Cys Phe Ser Val Gln Ala Val Ile Pro Ser Arg Thr Val Asn
       185                 190                 195
   CGG AAG AGT ACA GAC AGC CCG GTA GAG TGT ATG GGC CAG GAG AAA GGG        774
   Arg Lys Ser Thr Asp Ser Pro Val Glu Cys Met Gly Gln Glu Lys Gly
   200                 205                 210                 215
   GAA TTT AGA GAA TAA GAATTC                                             795
   Glu Phe Arg Glu  *
                   220
(2) INFORMATION FOR SEQ ID NO:19:
   (i) SEQUENCE CHARACTERISTICS:
      (A) LENGTH: 251 amino acids
      (B) TYPE: amino acid
      (D) TOPOLOGY: linear
   (ii) MOLECULE TYPE: protein
   (xi) SEQUENCE DESCRIPTION: SEQ ID NO:19:
   Met Glu Thr Pro Ala Trp Pro Arg Val Pro Arg Pro Glu Thr Ala Val
   -32     -30                 -25                 -20
   Ala Arg Thr Leu Leu Leu Gly Trp Val Phe Ala Gln Val Ala Gly Ala
       -15                 -10                 -5
   Ser Gly Thr Thr Asn Thr Val Ala Ala Tyr Asn Leu Thr Trp Lys Ser
    1              5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu  *
       210                 215                 220
(2) INFORMATION FOR SEQ ID NO:20:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:20:
   Ser Gly Thr Thr Asn Thr Val Ala Ala Tyr Asn Leu Thr Trp Lys Ser
     1               5                  10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Gln Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:21:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:21:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Ala Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:22:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:22:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Glu Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:23:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:23:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Lys Gln Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu
   210 215
(2) INFORMATION FOR SEQ ID NO:24:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:24:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
   50 55 60
   Lys Asp Val Lys Gln Thr Tyr Leu Ala Arg Val Phe Ser Tyr Pro Ala
   65 70 75 80
   Gly Asn Val Glu Ser Thr Gly Ser Ala Gly Glu Pro Leu Tyr Glu Asn
   85 90 95
   Ser Pro Glu Phe Thr Pro Tyr Leu Glu Thr Asn Leu Gly Gln Pro Thr
   100 105 110
   Ile Gln Ser Phe Glu Gln Val Gly Thr Lys Val Asn Val Thr Val Glu
   115 120 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Lys Ala Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu
   210 215
(2) INFORMATION FOR SEQ ID NO:25:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:25:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
   50 55 60
   Lys Asp Val Lys Gln Thr Tyr Leu Ala Arg Val Phe Ser Tyr Pro Ala
   65 70 75 80
   Gly Asn Val Glu Ser Thr Gly Ser Ala Gly Glu Pro Leu Tyr Glu Asn
   85 90 95
   Ser Pro Glu Phe Thr Pro Tyr Leu Glu Thr Asn Leu Gly Gln Pro Thr
   100 105 110
   Ile Gln Ser Phe Glu Gln Val Gly Thr Lys Val Asn Val Thr Val Glu
   115 120 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Lys Glu Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu
   210 215
(2) INFORMATION FOR SEQ ID NO:26:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:26:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Gln Gln Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:27:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:27:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Gln Ala Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:28:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:28:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Ala Gln Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:29:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:29:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Gln Glu Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:30:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:30:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Glu Gln Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu
   210 215
(2) INFORMATION FOR SEQ ID NO:31:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:31:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
   50 55 60
   Lys Asp Val Lys Gln Thr Tyr Leu Ala Arg Val Phe Ser Tyr Pro Ala
   65 70 75 80
   Gly Asn Val Glu Ser Thr Gly Ser Ala Gly Glu Pro Leu Tyr Glu Asn
   85 90 95
   Ser Pro Glu Phe Thr Pro Tyr Leu Glu Thr Asn Leu Gly Gln Pro Thr
   100 105 110
   Ile Gln Ser Phe Glu Gln Val Gly Thr Lys Val Asn Val Thr Val Glu
   115 120 125
   Asp Glu Arg Thr Leu Val Arg Arg Asn Asn Thr Phe Leu Ser Leu Arg
   130 135 140
   Asp Val Phe Gly Lys Asp Leu Ile Tyr Thr Leu Tyr Tyr Trp Lys Ser
   145 150 155 160
   Ser Ser Ser Gly Ala Ala Thr Ala Lys Thr Asn Thr Asn Glu Phe Leu
   165 170 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu
   210 215
(2) INFORMATION FOR SEQ ID NO:32:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:32:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Ala Glu Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:33:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:33
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Glu Ala Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:34:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (xi) SEQUENCE DESCRIPTION: SEQ ID NO:34:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Glu Glu Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:35:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (ix) FEATURE:
      (A) NAME/KEY: Modified-site
      (B) LOCATION: 165
      (D) OTHER INFORMATION: /note= "AMINO ACID AT POSITION 165 LABELED AS Xaa CAN BE ANY AMINO ACID EXCEPT LYSINE (LYS OR K)"
   (xi) SEQUENCE DESCRIPTION: SEQ ID NO:35:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Xaa Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
   180 185 190
   Ile Pro Ser Arg Thr Val Asn Arg Lys Ser Thr Asp Ser Pro Val Glu
   195 200 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser
   260
(2) INFORMATION FOR SEQ ID NO:36:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (ix) FEATURE:
      (A) NAME/KEY: Modified-site
      (B) LOCATION: 166
      (D) OTHER INFORMATION: /note= "AMINO ACID AT POSITION 166 LABELED AS Xaa CAN BE ANY AMINO ACID EXCEPT LYSINE (LYS OR K)"
   (xi) SEQUENCE DESCRIPTION: SEQ ID NO:36:
   Ser Gly Thr Thr Asn Thr Val Ala Ala Tyr Asn Leu Thr Trp Lys Ser
   1 5 10 15
   Thr Asn Phe Lys Thr Ile Leu Glu Trp Glu Pro Lys Pro Val Asn Gln
   20 25 30
   Val Tyr Thr Val Gln Ile Ser Thr Lys Ser Gly Asp Trp Lys Ser Lys
   35 40 45
   Cys Phe Tyr Thr Thr Asp Thr Glu Cys Asp Leu Thr Asp Glu Ile Val
   50 55 60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Xaa Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                 240
   Ser Leu His Lys Cys Arg Lys Ala Gly Val Gly Gln Ser Trp Lys Glu
                   245                 250                 255
   Asn Ser Pro Leu Asn Val Ser
               260
(2) INFORMATION FOR SEQ ID NO:37:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (ix) FEATURE:
      (A) NAME/KEY: Modified-site
      (B) LOCATION: 165..166
      (D) OTHER INFORMATION: /note= "AMINO ACIDS AT POSITION 165 AND 166 LABELED AS Xaa CAN BE ANY AMINO ACID EXCEPT LYSINE (LYS OR K)"
   (xi) SEQUENCE DESCRIPTION: SEQ ID NO:37:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Xaa Xaa Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                 240
   Ser Leu His Lys Cys Arg Lys Ala Gly Val Gly Gln Ser Trp Lys Glu
                   245                 250                 255
   Asn Ser Pro Leu Asn Val Ser
               260
(2) INFORMATION FOR SEQ ID NO:38:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (ix) FEATURE:
      (A) NAME/KEY: Modified-site
      (B) LOCATION: 165
      (D) OTHER INFORMATION: /note= "AMINO ACID AT POSITION 165 LABELED AS Xaa CAN BE ANY AMINO ACID EXCEPT LYSINE (LYS OR K)"
   (xi) SEQUENCE DESCRIPTION: SEQ ID NO:38:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Xaa Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:39:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (ix) FEATURE:
      (A) NAME/KEY: Modified-site
      (B) LOCATION: 166
      (D) OTHER INFORMATION: /note= "AMINO ACID AT POSITION 166 LABELED AS Xaa CAN BE ANY AMINO ACID EXCEPT LYSINE (LYS OR K)"
   (xi) SEQUENCE DESCRIPTION: SEQ ID NO:39:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Xaa Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:40:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (ix) FEATURE:
      (A) NAME/KEY: Modified-site
      (B) LOCATION: 165..166
      (D) OTHER INFORMATION: /note= "AMINO ACIDS AT POSITION 165 AND 166 LABELED AS Xaa CAN BE ANY AMINO ACID EXCEPT LYSINE (LYS OR K)"
   (xi) SEQUENCE DESCRIPTION: SEQ ID NO:40:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Xaa Xaa Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu
       210                 215
(2) INFORMATION FOR SEQ ID NO:41:
   (i) SEQUENCE CHARACTERISTICS:
      (A) LENGTH: 263 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (ix) FEATURE:
      (A) NAME/KEY: Modified-site
      (B) LOCATION: 154..204
      (D) OTHER INFORMATION: /note= "AT LEAST ONE OR MORE OF THE AMINO ACIDS LABELED AS Xaa ARE MUTATED FROM THE AMINO ACID AT THAT POSITION IN WILD TYPE TISSUE FACTOR SHOWN IN SEQ ID NO:1"
   (xi) SEQUENCE DESCRIPTION: SEQ ID NO:41:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
   130 135 140
   Asp Val Phe Gly Lys Asp Leu Ile Tyr Xaa Leu Tyr Tyr Trp Xaa Ser
   145 150 155 160
   Ser Ser Xaa Xaa Xaa Xaa Thr Ala Lys Thr Asn Thr Asn Glu Phe Leu
   165 170 175
   Ile Xaa Val Xaa Lys Gly Glu Asn Tyr Cys Phe Ser Val Gln Ala Val
   180 185 190
   Ile Pro Ser Arg Thr Val Asn Arg Xaa Ser Thr Xaa Ser Pro Val Glu
   195 200 205
   Cys Met Gly Gln Glu Lys Gly Glu Phe Arg Glu Ile Phe Tyr Ile Ile
   210 215 220
   Gly Ala Val Val Phe Val Val Ile Ile Leu Val Ile Ile Leu Ala Ile
   225 230 235 240
   Ser Leu His Lys Cys Arg Lys Ala Gly Val Gly Gln Ser Trp Lys Glu
   245 250 255
   Asn Ser Pro Leu Asn Val Ser
   260
(2) INFORMATION FOR SEQ ID NO:42:
   (i) SEQUENCE CHARACTERISTICS:
      (A) LENGTH: 219 amino acids
      (B) TYPE: amino acid
      (C) STRANDEDNESS: not relevant
      (D) TOPOLOGY: not relevant
   (ii) MOLECULE TYPE: protein
   (vi) ORIGINAL SOURCE:
      (A) ORGANISM: Homo sapiens
   (ix) FEATURE:
      (A) NAME/KEY: Modified-site
      (B) LOCATION: 154..204
      (D) OTHER INFORMATION: /note= "AT LEAST ONE OR MORE OF THE AMINO ACIDS LABELED AS Xaa ARE MUTATED FROM THE AMINO ACID AT THAT POSITION IN THE TRUNCATED TISSUE FACTOR SHOWN IN SEQ ID NO:18"
   (xi) SEQUENCE DESCRIPTION: SEQ ID NO:42:
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Xaa Leu Tyr Tyr Trp Xaa Ser
   145                 150                 155                 160
   Ser Ser Xaa Xaa Xaa Xaa Thr Ala Lys Thr Asn Thr Asn Glu Phe Leu
                   165                 170                 175
   Ile Xaa Val Xaa Lys Gly Glu Asn Tyr Cys Phe Ser Val Gln Ala Val
               180                 185                 190
   Ile Pro Ser Arg Thr Val Asn Arg Xaa Ser Thr Xaa Ser Pro Val Glu
           195                 200                 205
   Cys Met Gly Gln Glu Lys Gly Glu Phe Arg Glu
       210                 215
(2) INFORMATION FOR SEQ ID NO:43:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:43:
   TCAAGTTCAG GAGCGGCGAC AGCCAAAACA 30
(2) INFORMATION FOR SEQ ID NO:44: .
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:44:
   CGCCGCTCCT GAACTTGAAG ATTTCC 26
(2) INFORMATION FOR SEQ ID NO:45:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:45:
   TCAAGTTCAG GAGAAGAAAC AGCCAAAACA 30
(2) INFORMATION FOR SEQ ID NO:46:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:46:
   GTTTCTTCTC CTGAACTTGA AGATTTCC 28
(2) INFORMATION FOR SEQ ID NO:47:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:47:
   TCAAGTTCAG GACAGCAGAC AGCCAAAACA                                    30
(2) INFORMATION FOR SEQ ID NO:48:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:48:
   TCTGCTGTCC TGAACTTGAA GATTTCC                                       27

## Description

### TECHNICAL FIELD OF THE INVENTION

This invention relates to the field of detection of blood clotting factors VIII and IX.

### BACKGROUND OF THE INVENTION

Currently, the most widely used clotting assays for measuring the overall state of the plasma clotting factors are the Prothrombin Time test (PT test) and the activated partial thrombin plastin time test (aPTT). Both the PT test and the aPTT test are based on crude activators of clotting. Even the thromboplastin reagents based on recombinant Tissue Factor (TF) are made to imitate crude brain extracts in terms of their clot-promoting ability. It has been recently disclosed by Morrissey that recombinant soluble tissue factor is useful in an assay for specifically measuring activated factor VII ("factor VIIa" or "FVIIa"). U.S. Patent No. 5,472,850.

The Prothrombin Time test (PT test) is a widely used clinical test of the blood clotting system. The PT test is typically performed on citrated plasma samples by adding thromboplastin and a source of calcium ions. The elapsed time between the addition of calcium ions and detection of clot formation is measured. Thromboplastin typically refers to crude tissue extracts (brain, lung and/or placenta) that contain tissue factor activity. More recently, recombinant human tissue factor (reconstituted into phospholipid vesicles) has become available as a replacement for crude thromboplastin preparations. The amount of thromboplastin added in such assays is adjusted so that normal plasma samples will typically form a clot in about 12 to 15 seconds. The actual times will depend to some extent on the method used to detect clot formation. PT assays are routinely used as general screening tests for abnormalities of clotting proteins (hereditary and acquired) and to monitor oral anticoagulant therapy. A prolonged clotting time may be indicative of a coagulation disorder or of effectiveness of anticoagulant therapy.

The PT test is dependent upon the following clotting factors being present in the plasma to be tested: factors VII, X, V, prothrombin, and fibrinogen. If any of these factors are missing or seriously deficient, the PT will be prolonged. Oral anticoagulant therapy (coumadin and its relatives) antagonizes the action of vitamin K and depresses the levels of vitamin K-dependent proteins in plasma. The vitamin K-dependent proteins that influence the PT are factors VII, X and prothrombin.

There are two additional clotting factors that are necessary for normal hemostasis but not measured by the PT test: factor VIII ("FVIII") and factor IX ("FIX"). It has been reported that highly dilute thromboplastin will result in a PT test with somewhat increased sensitivity to Factors VIII and IX, but this is seldom used because the clotting times are unrealistically long. In order to test for these factors, another routine clotting test, known as the activated partial thromboplastin time (aPTT) is performed. The aPTT test is typically performed on citrated plasma samples by adding aPTT reagent and a source of calcium ions. The elapsed time between the addition of calcium ions and detection of clot formation is measured. Typically, aPTT reagents are composed of a mixture of phospholipid vesicles (cephalin) and an activator of the contact pathway of clotting. Commonly used activators are kaolin or ellagic acid.

The aPTT test depends on the presence of the following clotting factors in plasma: factors XI, XII, VIII, IX, X, V, prothrombin, and fibrinogen. In addition to screening for deficiencies in factors VIII and IX, the aPTT test is often used to monitor heparin therapy and to screen for lupus anticoagulants. Deficiency in factor XI is rarely associated with bleeding tendencies, while deficiencies in factor XII or any of the other upstream members of the contact pathway cause no bleeding tendency whatever. Therefore, prolongation of the aPTT may mean that the individual has a clinically relevant clotting abnormality, or, in contrast, it may mean that the individual has clinically unimportant deficiency in one of the contact factors.

Prolongations of either the PT or aPTT can be followed up with assays of specific clotting factors to determine if a factor deficiency is responsible. They can also be followed up by specialized tests to determine if inhibitors of clotting (such as lupus anticoagulants, heparin, etc.) are responsible.

However, there remains a continuing need for an assay specific for other clotting factors known to be necessary for normal hemostasis. Specifically, there remains a need for an assay to measure factors VIII and IX directly so as to eliminate the need for two or more clotting assays in routine screening (PT + aPTT). A direct assay would eliminate the problem caused by the fact that the aPTT is sensitive to several plasma factors that do not actually participate in normal hemostasis.

It is also desirable to alter the sensitivity of the PT assay to warfarin or heparin treatment.

Mutation of two lysine residues in tissue factor (residues 165 and 166) to alanine was reported by Roy et al. to greatly reduce the ability of the resulting tissue factor-factor VIIa complex to activate factor X. Roy, et al., *J Biol Chem* 266:22063-22066 (1991). Subsequently, Ruf et al. made the same mutations and reported the complex of mutant tissue factor-factor VIIa had an increased K_{*m*} for factor X activation. They concluded that either these residues bind to factor X, or they bind to factor VIIa and influence its affinity for factor X. Ruf, et al., *J Biol Chem* 267:6375-6381 (1992a). A later paper by Ruf et al. mutated both these two residues and some surrounding residues to alanine. They showed that several different mutations in this region caused the resulting tissue factor-factor VIIa complex to have reduced activation of factor X. Ruf et al. also reported results of some clotting assays with factor IX-deficient plasma. Ruf, et al., *J Biol Chem* 267:22206-22210 (1992b). In International Patent Application WO 94/07515, Ruf et al. discloses a composition comprising mutant human tissue factor having the amino acid at position 165 and 166 substituted with alanine and a method for detecting the amount of factor VIIa in a body fluid using various forms of mutant human tissue factor, one of which had the substitution of alanine at position 165 and 166.

A specific assay for Factor VIII and Factor IX has now been found using genetically altered tissue factor mutated at position 165 and/or 166. Mutating lysines 165 and 166 of tissue factor to alanine (A), glutamine(Q) or glutamic acid (E) greatly reduces the ability of the resulting tissue factor-factor VIIa complex to activate factor X. The different substitutions have different effects on factor X activation. In order of decreasing severity they were: glutamic acid > alanine > glutamine. These same mutations decrease the ability of the resulting tissue factor-factor VIIa complex to activate factor IX as well. However, the severity of the effect is much less pronounced than with factor X. This is particularly true of the tissue factor mutants in which lysines 165 and 166 are replaced by glutamine (TF-Q¹⁶⁵Q¹⁶⁶).

Further, the clotting times of TF-Q¹⁶⁵Q¹⁶⁶ is significantly prolonged in factor VIII- or factor IX-deficient plasma under conditions where the clotting time of wild-type TF is almost the same in normal versus factor VIII- or IX-deficient plasma. Therefore, a PT-like clotting assay that measures all the clotting factors known to be associated with bleeding tendencies is now disclosed.

### BRIEF DESCRIPTION OF DRAWINGS

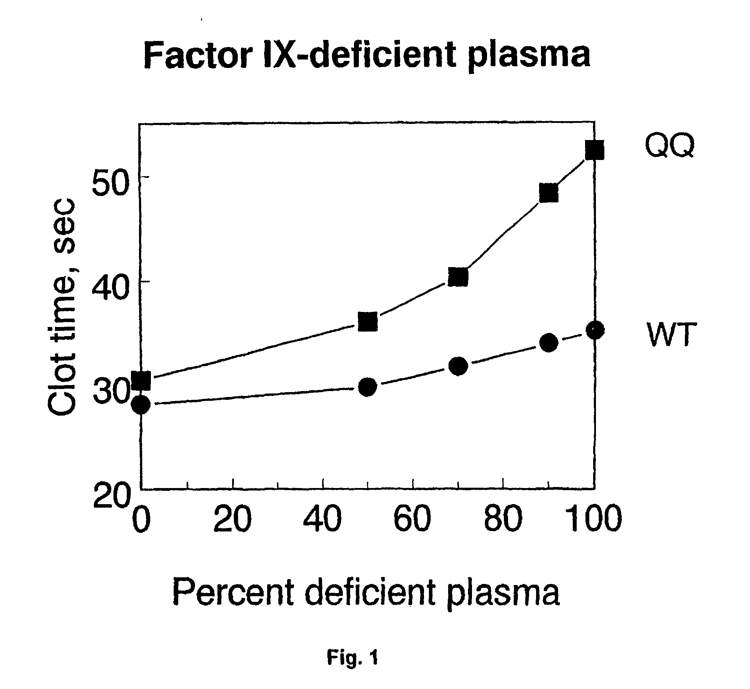

Fig. 1 is a line graph depicting the clotting times of TF-Q¹⁶⁵Q¹⁶⁶ (squares) and wild-type TF (circles) in a clotting assay using Factor IX-deficient plasma mixed together in varying proportions with normal plasma (indicated on x-axis as "Percent deficient plasma"). The clotting times were measured at 37°C by mixing together 50 µl TF solution, 50 µl citrated human plasma (consisting of varying mixtures of normal and Factor IX-deficient plasma), and 50 µl of 25 mM calcium chloride solution.
The time to clot formation was measured from the time of addition of the calcium chloride solution. For wild-type TF, the TF solution consisted of 14 ng/ml TF reconstituted into vesicles composed of 20% phosphatidyl serine and 80% phosphatidyl choline. For TF Q¹⁶⁵Q¹⁶⁶, the TF solution consisted of 1 µg/ml TF Q¹⁶⁵Q¹⁶⁶ reconstituted into vesicles composed of 20% phosphatidyl serine and 80% phosphatidyl choline.

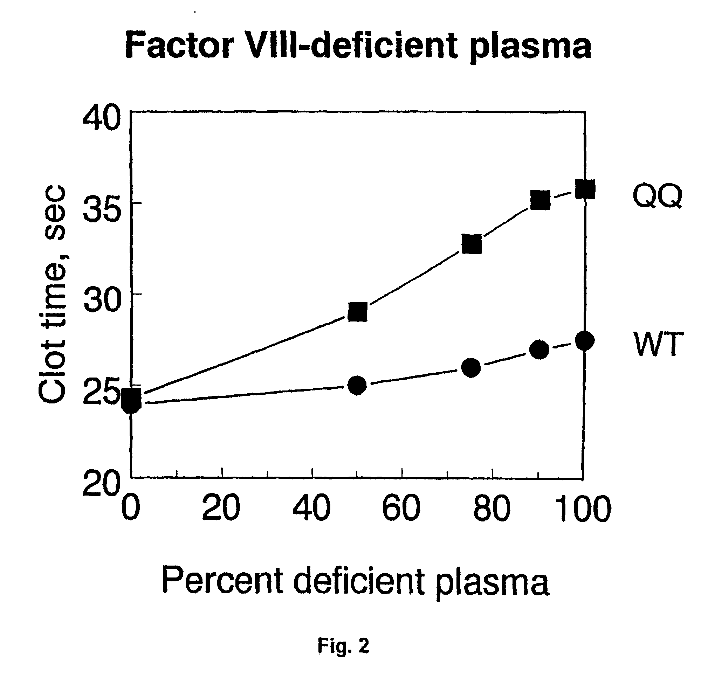

Fig. 2 is a line graph depicting the clotting times of TF-Q¹⁶⁵Q¹⁶⁶ (squares) and wild-type TF (circles) in a clotting assay using Factor VIII-deficient plasma mixed together in varying proportions with normal plasma (indicated on x-axis as "Percent deficient plasma"). The clotting times were measured at 37°C by mixing together 50 µl TF solution, 50 µl citrated human plasma (consisting of varying mixtures of normal and Factor VIII-deficient plasma), and 50 µl of 25 mM calcium chloride solution. The time to clot formation was measured from the time of addition of the calcium chloride solution. For wild-type TF, the TF solution consisted of 14 ng/ml TF reconstituted into vesicles composed of 20% phosphatidyl serine and 80% phosphatidyl choline. For TF Q¹⁶⁵Q¹⁶⁶, the TF solution consisted of 1 µg/ml TF Q¹⁶⁵Q¹⁶⁶ reconstituted into vesicles composed of 20% phosphatidyl serine and 80% phosphatidyl choline.

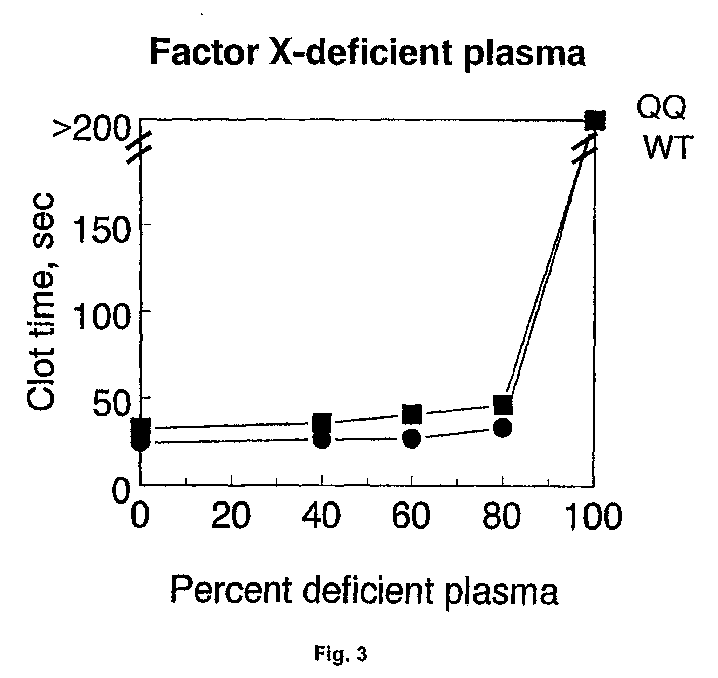

Fig. 3 is a line graph depicting the clotting times of TF-Q¹⁶⁵Q¹⁶⁶ (squares) and wild-type TF (circles) in a clotting assay using Factor X-deficient plasma mixed together in varying proportions with normal plasma (indicated on x-axis as "Percent deficient plasma"). The clotting times were measured at 37°C by mixing together 50 µl TF solution, 50 µl citrated human plasma (consisting of varying mixtures of normal and Factor X-deficient plasma), and 50 µl of 25 mM calcium chloride solution. The time to clot formation was measured from the time of addition of the calcium chloride solution. For wild-type TF, the TF solution consisted of 14 ng/ml TF reconstituted into vesicles composed of 20% phosphatidyl serine and 80% phosphatidyl choline. For TF Q¹⁶⁵Q¹⁶⁶, the TF solution consisted of 1 µg/ml TF Q¹⁶⁵Q¹⁶⁶ reconstituted into vesicles composed of 20% phosphatidyl serine and 80% phosphatidyl choline.

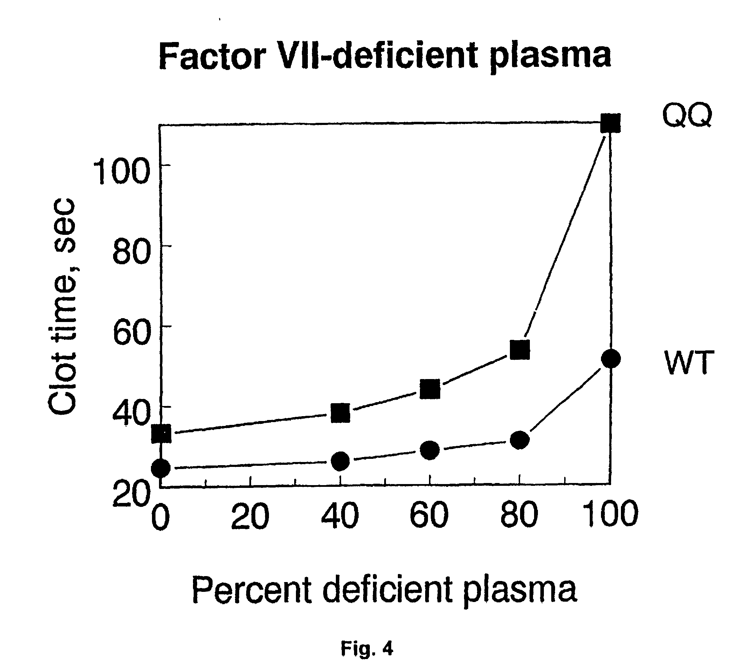

Fig. 4 is a line graph depicting the clotting times of TF-Q¹⁶⁵Q¹⁶⁶ (squares) and wild-type TF (circles) in a clotting assay using Factor VII-deficient plasma mixed together in varying proportions with normal plasma (indicated on x-axis as "Percent deficient plasma"). The clotting times were measured at 37°C by mixing together 50 µl TF solution, 50 µl citrated human plasma (consisting of varying mixtures of normal and Factor VII-deficient plasma), and 50 µl of 25 mM calcium chloride solution. The time to clot formation was measured from the time of addition of the calcium chloride solution. For wild-type TF, the TF solution consisted of 14 ng/ml TF reconstituted into vesicles composed of 20% phosphatidyl serine and 80% phosphatidyl choline. For TF Q¹⁶⁵Q¹⁶⁶, the TF solution consisted of 1 µg/ml TF Q¹⁶⁵Q¹⁶⁶ reconstituted into vesicles composed of 20% phosphatidyl serine and 80% phosphatidyl choline.

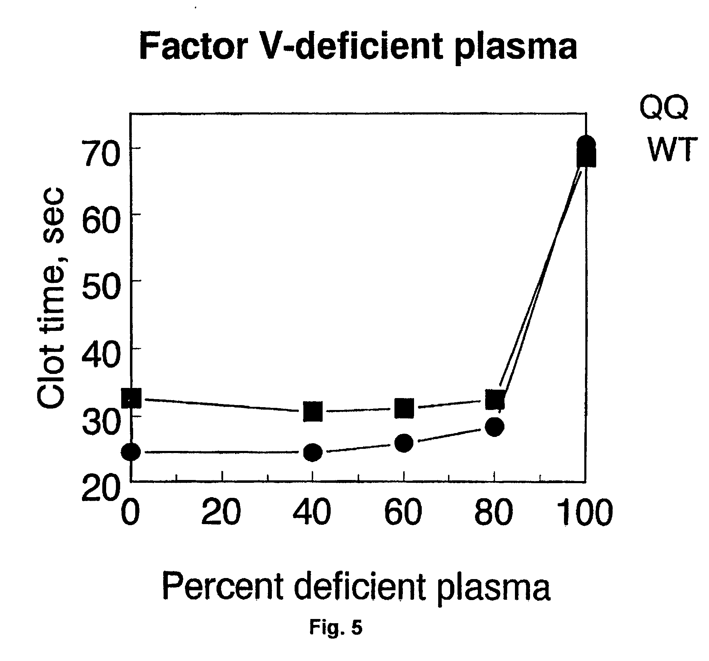

Fig. 5 is a line graph depicting the clotting times of TF-Q¹⁶⁵Q¹⁶⁶ (squares) and wild-type TF (circles) in a clotting assay using Factor V-deficient plasma mixed together in varying proportions with normal plasma (indicated on x-axis as "Percent deficient plasma"). The clotting times were measured at 37°C by mixing together 50 µl TF solution, 50 µl citrated human plasma (consisting of varying mixtures of normal and Factor V-deficient plasma), and 50 µl of 25 mM calcium chloride solution. The time to clot formation was measured from the time of addition of the calcium chloride solution. For wild-type TF, the TF solution consisted of 14 ng/ml TF reconstituted into vesicles composed of 20% phosphatidyl serine and 80% phosphatidyl choline. For TF Q¹⁶⁵Q¹⁶⁶, the TF solution consisted of 1 µg/ml TF Q¹⁶⁵Q¹⁶⁶ reconstituted into vesicles composed of 20% phosphatidyl serine and 80% phosphatidyl choline.

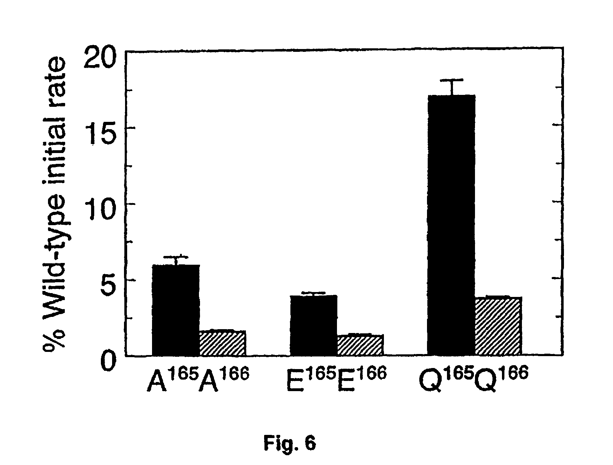

Fig. 6 is a bar graph depicting FX activation by TF-FVIIa. For assays with TF relipidated in a mixture of 80% phosphatidyl choline in 20% phosphatidyl serine (PCPS) vesicles (closed bars), reaction conditions were: 150 nM FX, 1 nM TF in 10-14 µM PCPS, 10 pM FVIIa. For assays with TF relipidated in pure phosphatidyl choline ("PC") vesicles (hatched bars), reaction conditions were: 1 µM FX, 1 nM TF in 9-13 µM PC, 100 pM FVIIa. Values represent the mean initial rates of FX activation, normalized to the rate observed with wild-type TF (100% = 319 ±7 M/min/M and 100% = 41 ± 0.7 M/min/M for wild-type TF in PCPS and PC, respectively). Error bars denote SEM calculated for three independent experiments.

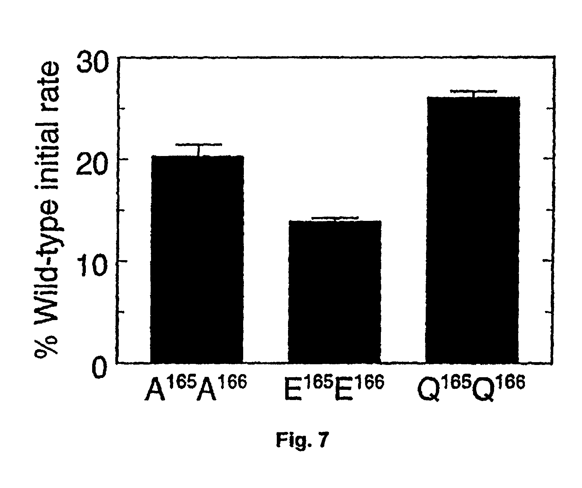

Fig. 7 is a bar graph depicting FIX activation by TF-FVIIa. Reaction conditions were: 150 nM FIX, 1 nM TF in 10-14 µM PCPS, 50 pM FVIIa. Values represent the mean initial rates of FIX activation, normalized to the rate observed with wild-type TF (100% = 127 ± 7 M/min/M).
Error bars denote SEM calculated for three independent experiments.

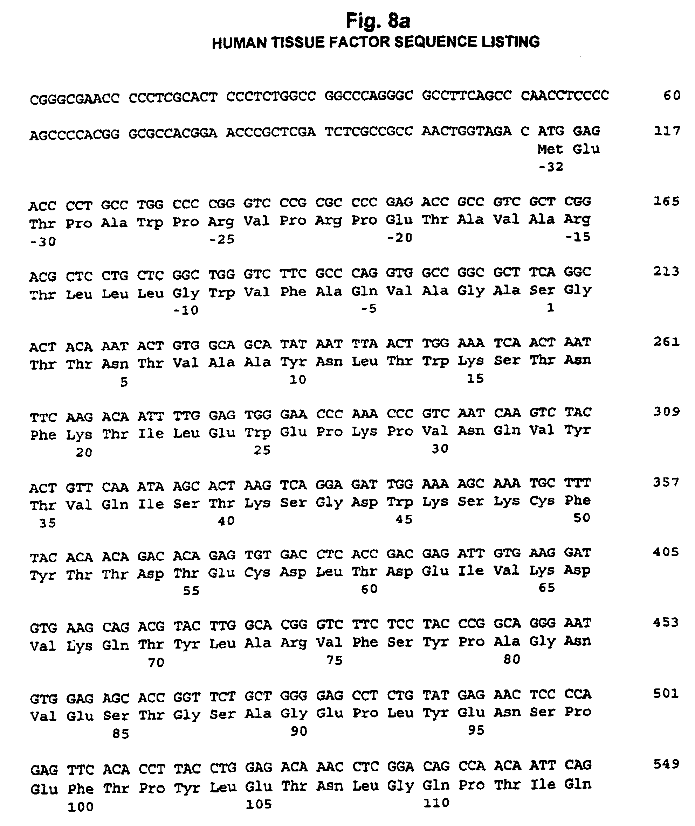

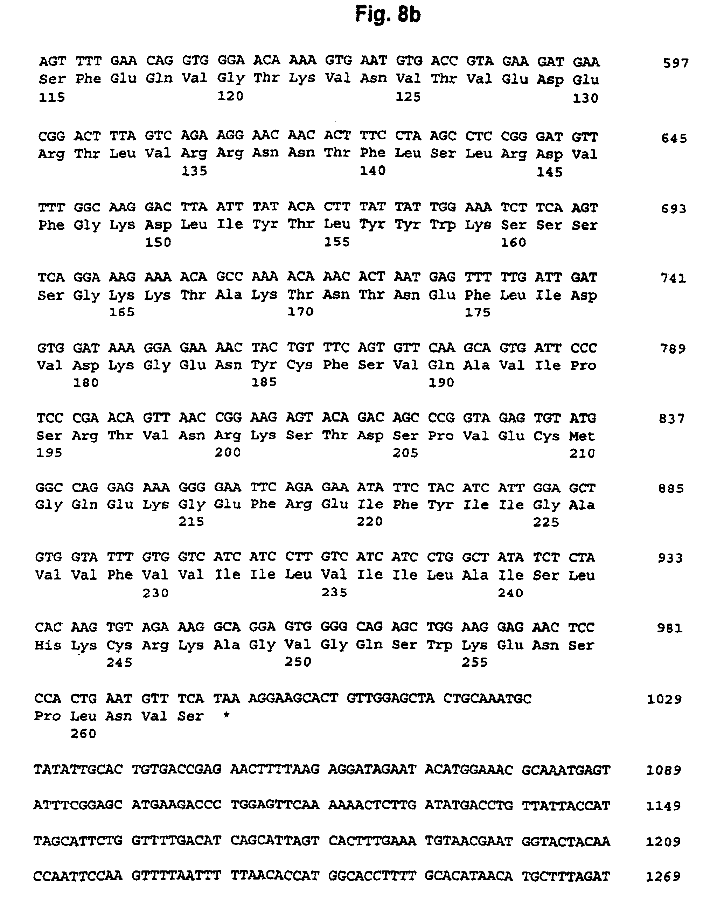

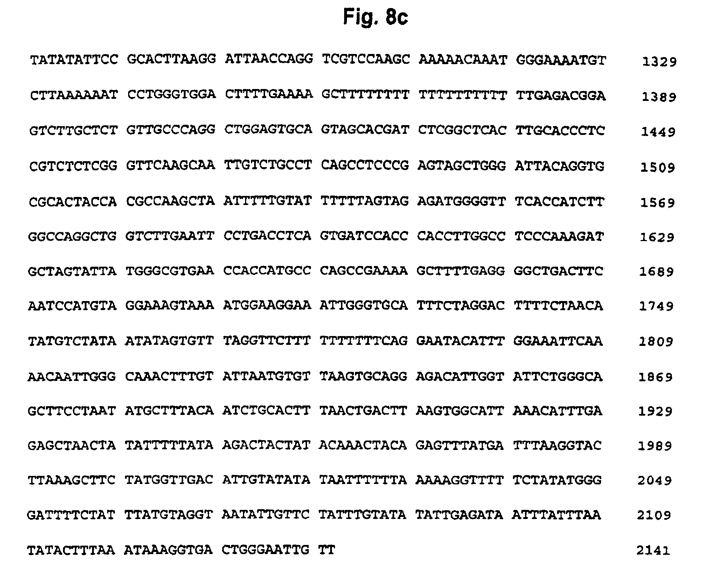

Fig. 8a - Fig. 8c is the sequence listing for wild-type TF.

### DETAILED DESCRIPTION

An assay for determining Factor VIII or IX deficiency is now reported.

In the practice of the invention, a form of Tissue Factor (TF) is employed which has been mutated ("mutated tissue factor"), preferably in position 165 and 166 (hereinafter referred to as TF^{165,166}). Native, or wild type, TF (nucleic acid sequence, SEQ ID NO:1; amino acid sequence, SEQ ID NO:2) contains the amino acid lysine (Lys or K) in these positions. In practicing the invention, mutations of both residues K¹⁶⁵ and K¹⁶⁶ of TF to alanine (Ala or A), glutamic acid (Glu or E) or glutamine (Gln or Q) (K¹⁶⁵ to Q¹⁶⁵ = SEQ ID NO:3; K¹⁶⁵ to A¹⁶⁵ = SEQ ID NO:4; K¹⁶⁵ to E¹⁶⁵ = SEQ ID NO:5; K¹⁶⁶ to Q¹⁶⁶ = SEQ ID NO:6; K¹⁶⁶ to A¹⁶⁶ = SEQ ID NO:7; K¹⁶⁶ to E¹⁶⁶ = SEQ ID NO:8; K¹⁶⁵K¹⁶⁶ to Q¹⁶⁵Q¹⁶⁶ = SEQ ID NO:9; K¹⁶⁵K¹⁶⁶ to Q¹⁶⁵A¹⁶⁶ = SEQ ID NO:10; K¹⁶⁵K¹⁶⁶ to A¹⁶⁵Q¹⁶⁶ = SEQ ID NO:11; K¹⁶⁵K¹⁶⁶ to Q¹⁶⁵E¹⁶⁶ = SEQ ID NO:12; K¹⁶⁵K¹⁶⁶ to E¹⁶⁵Q¹⁶⁶ = SEQ ID NO:13; K¹⁶⁵K¹⁶⁶ to A¹⁶⁵A¹⁶⁶ = SEQ ID NO:14; K¹⁶⁵K¹⁶⁶ to A¹⁶⁵E¹⁶⁶ = SEQ ID NO:15; K¹⁶⁵K¹⁶⁶ to E¹⁶⁵A¹⁶⁶ = SEQ ID NO:16; and K¹⁶⁵K¹⁶⁶ to E¹⁶⁵E¹⁶⁶ = SEQ ID NO:17) are effective to support FVIIa-mediated activation of FX and factor IX (FIX). Each TF mutant is equivalent to wild-type TF in both FVIIa binding and promotion of FVIIa amidolytic activity but markedly deficient in supporting FIX and FX activation. Specifically, for Q¹⁶⁵Q¹⁶⁶, A¹⁶⁵A¹⁶⁶, and E¹⁶⁵E¹⁶⁶, it has been shown that all three mutants are markedly deficient in supporting FIX and FX activation, with FX activation rates decreased more than FIX activation rates. In both reactions, the TF mutants exhibit different extents of activity: Q¹⁶⁵Q¹⁶⁶ > A¹⁶⁵A¹⁶⁶ > E¹⁶⁵E¹⁶⁶, TF^{165,166} can be made according to the method of Example 1.

TF, an integral membrane protein, is the essential protein cofactor for the plasma serine protease, FVIIa. The cell-surface complex of TF and FVIIa (TF-FVIIa) is the first enzyme in the clotting cascade and is responsible for initiating blood coagulation both in normal hemostasis and in thrombotic disease. Carson, S.D. and Brozna, J.P., *Blood Coag Fibrinol* 4:281-292 (1993) The natural substrates for the TF.FVIIa complex are the serine protease zymogens, FVII, FIX, and FX.

The altered or "mutated tissue factor" of the invention may be mutated full length tissue factor or mutated truncated tissue factor. Reported truncated tissue factors include those which do not include all of the transmembrane domain of wild-type tissue factor and those which do not include all of the cytoplasmic domain of wild type tissue factor. The membrane-spanning domain has been previously identified as spanning amino acids 220-242. Morrissey et al., "Molecular cloning of the cDNA for tissue factor, the cellular receptor for the initiation of the coagulation protease cascade," *Cell* 50:129-135 (1987). Soluble tissue factor having amino acids 1-219 (nucleic acid sequence, SEQ ID NO: 18 and amino acid sequence, SEQ ID NO:19) was utilized in an assay for activated Factor VII in Morrissey et al., *Blood* 81(3): 734-744. Recombinant tissue factor having amino acids 1-243, lacking the cytoplasmic tail, was described by Paborsky et al., *J Biol Chem* 266:21911-16 (1991). The length of the mutated tissue factor is not believed to be important, and it is envisioned that truncated forms of tissue factor described in the literature, with the further mutations at either position 165, 166, or preferably both, will work in the assay of the invention, e.g., soluble tissue factor SEQ ID NO:18 having mutations at K¹⁶⁵ or K¹⁶⁶, or both to Q, A, or E (K¹⁶⁵ to Q¹⁶⁵ = SEQ ID NO:20; K¹⁶⁵ to A¹⁶⁵ = SEQ ID NO:21; K¹⁶⁵ to E¹⁶⁵ = SEQ ID NO:22; K¹⁶⁶ to Q¹⁶⁶ = SEQ ID NO:23; K¹⁶⁶ to A¹⁶⁶ = SEQ ID NO:24; K¹⁶⁶ to E¹⁶⁶ = SEQ ID NO:25; K¹⁶⁵K¹⁶⁶ to Q¹⁶⁵Q¹⁶⁶ = SEQ ID NO:26; K¹⁶⁵K¹⁶⁶ to Q¹⁶⁵A¹⁶⁶ = SEQ ID NO:27; K¹⁶⁵K¹⁶⁶ to A¹⁶⁵Q¹⁶⁶ = SEQ ID NO:28; K¹⁶⁵K¹⁶⁶ to Q¹⁶⁵E¹⁶⁶ = SEQ ID NO:29; K¹⁶⁵K¹⁶⁶ to E¹⁶⁵Q¹⁶⁶ = SEQ ID NO:30; K¹⁶⁵K¹⁶⁶ to A¹⁶⁵A¹⁶⁶ = SEQ ID NO:31; K¹⁶⁵K¹⁶⁶ to A¹⁶⁵E¹⁶⁶ = SEQ ID NO:32; K¹⁶⁵K¹⁶⁶ to E¹⁶⁵A¹⁶⁶ = SEQ ID NO:33; and K¹⁶⁵K¹⁶⁶ to E¹⁶⁵E¹⁶⁶ = SEQ ID NO:34). Further, it is envisioned that minor variations in length from the truncated forms described in the literature (plus or minus two to five amino acids) will not affect the activity. For example, for mutated truncated tissue factor, sequences can start from about amino acid about -5 to about 5 and end at about 214 to about 248 of SEQ ID NO:1. Likewise, for wild type tissue factor, sequences can start from about amino acid about -5 to about 5 and end at about 258 to about 268 of SEQ ID NO:1. An appropriate length can be tested for easily by using standard clotting assays with control plasma and determining that clotting occurs within a readily measured time frame, preferably 10 to 30 seconds.

The mutation of the tissue factor sequence selected can be done at position 165 or position 166 or preferably both position 165 and 166. It is believed that substitution of the original amino acid, lysine (lys or K), with any other amino acid will produce a reagent superior to thromboplastin reagents commonly used in clotting assays, e.g., for wild type tissue factor, substitution of K¹⁶⁵ in SEQ ID NO:35, of K¹⁶⁶ in SEQ ID NO:36, and of K¹⁶⁵K¹⁶⁶ in SEQ ID NO:37; and for truncated tissue factor, substitution of K¹⁶⁵ in SEQ ID NO:38, of K¹⁶⁶ in SEQ ID NO:39, and of K¹⁶⁵K¹⁶⁶ in SEQ ID NO:40. Preferably, one or both positions will be substituted with glutamine (gln or Q), alanine (ala or A) or glutamic acid (glu or E). The most preferable substitution is with the amino acid glutamine (abbreviated as gln or Q). The substitution at positions 165 and 166 is believed to be additive, so that it is most preferred that both positions are substituted with Q (i.e. QQ), but combinations such as for example, QA, AQ, QE, EQ, AA, AE, EA, EE and combinations with other amino acids other than Q, A, or E are believed to be suitable as well.

It is also believed that mutations at other positions of tissue factor can be accomplished to yield a tissue factor which has increased dependence on Factor IX in clotting assays. These are threonine at position 154, lysine at position 159, serine at position 163, glycine at position 164, aspartate at position 178, aspartate at position 180, lysine at position 201 and aspartate at position 204. It is believed that these positions can be altered by substituting any amino acid for the original, preferably Q, A or E, e.g., SEQ ID NO:41 for wild type tissue factor of SEQ ID NO:1 and SEQ ID NO:42 for soluble truncated tissue factor of SEQ ID NO:18. The methodology used to make these mutations would be by the basic protocol of Example 1, using modifications within the skill in the art to achieve the mutation at the alternate position.

The mutated tissue factor reagent useful in the present invention can be employed as a reagent in a clotting assay in substitution for thromboplastin reagents, whether from crude extracts or relipidated recombinant tissue factor. Commercially available thromboplastin reagents that the mutated tissue factor reagent could be used in place of are human thromboplastin, such as Thomborel S (Behringwerke AG, Marburg, Germany); human thromboplastin (recombinant), such as Recombiplastin (Ortho Diagnostic Systems) or Innovin (Baxter), rabbit thromboplastin, such as Thromboplastin, catalogue # T-0263 (Sigma Chemical Co., St. Louis Mo.). The procedures for such clotting assays, such as the PT and aPTT and modifications thereof are well known in the art. The reagent will also be useful in assays of the clotting employing hydrolysis of chromogenic or fluorogenic substrates as the endpoint instead of clotting. By using the mutated tissue factor reagent in such assays, the assays will be able to detect factor VIII and/or IX deficiencies. Membrane-anchored forms of mutated tissue factor would preferably be reconstituted into phospholipid vesicles before use. Soluble forms of mutated tissue factor would preferably be mixed with phospholipid vesicles before use.

The amount of mutated tissue factor to use in the assay is the amount effective to allow normal plasma to clot in a readily measured period in either an automated or manual PT assay. Preferably, the time of clotting with citrated normal human plasma will be less than about 2 minutes, preferably about seven seconds to about 2 minutes and most preferably about ten to thirty seconds. To readily determine the amount of reagent to use with unknown sample plasma, one could run a dose-response curve with normal control plasma and use the amount of mutated tissue factor which effects clotting of normal plasma within the time frame desired.

A numerical range of mutated tissue factor reagent to use would be from about 0.1 to about 50 micrograms per ml, more preferably 1 to about 20 micrograms per ml and most preferably about 15 micrograms per ml. The concentration can be adjusted as described in the previous paragraph to achieve the clotting time in the desired range for control plasma.

Although free FVIIa has limited enzymatic activity, binding to TF causes a large, reversible increase in its catalytic activity that can be detected with tripeptidylamide substrates as well as macromolecular substrates. Komiyama, et al., *Biochemistry* 29:9418-9425 (1990); Bom, V.J. and Bertina, R.M., *Biochem J* 265:327-336 (1990); Zur, M. and Nemerson, Y., *J Biol Chem* 253:2203-2209 (1978); Ruf, et al., *J Biol Chem* 266:2158-2166 (1991) and correction in *J Biol Chem* 266:16256 (1991); Higashi, et al, *J Biol Chem* 267:17990-17996 (1992); and Lawson, et al., *J Biol Chem* 267:4834-4843 (1992). The nature of the allosteric activation of FVIIa by TF (or of any blood coagulation serine protease by its cognate protein cofactor) is not well understood. The ability of TF to enhance the rate of cleavage of small amide and ester substrates by FVIIa indicates that it brings about changes in or near the catalytic center of FVIIa. However, TF might also contribute to the formation of an extended substrate binding site for recognition of the much larger macromolecular substrates. Indeed, factor Va is proposed to provide this function for its cognate protease, activated factor X ("FXa"), while thrombomodulin provides a similar function for protein C activation by thrombin. Mann, et al., *Ann Rev Biochem* 57:915-956 (1988).

Evidence has recently been obtained in support of the idea that TF may interact directly with macromolecular substrates. An initial study reported that chemical derivitization or alanine mutagenesis of two lysine residues K¹⁶⁵ and K¹⁶⁶ on TF greatly reduced the ability of the resulting TF-FVIIa complex to activate FX. Roy, et al., *J Biol Chem* 266:22063-22066 (1991). Mutations at K¹⁶⁵ or K¹⁶⁶ had additive effects, with the double mutant K¹⁶⁵ K¹⁶⁶ → A¹⁶⁵ A¹⁶⁶ having a greater defect in FX activation than either mutation alone. Interestingly, the mutations were without effect on the affinity of FVIIa for TF, or enhancement of FVIIa amidolytic activity by TF. Because the defect in FX activation of single mutations at either site could be partially overcome by increasing the phosphatidyl serine ("PS") content of the vesicles into which TF had been reconstituted, these investigators proposed that the two lysine residues may indirectly affect FX activation via interaction with negatively-charged phospholipid head groups. In a subsequent study by another group, it was demonstrated that mutating these two residues to alanine increased the apparent Kₘ for FX activation. Ruf, et al., *J Biol Chem* 267:6375-6381 (1992a). Furthermore, they showed that the defect in FX activation did not require interaction between TF and phospholipid head groups, since the phenomenon was observed even when complexes of FVIIa and soluble TF were used. It was therefore proposed that K¹⁶⁵ and K¹⁶⁶ may be involved in recognizing FX as a substrate, either directly by binding to FX, or indirectly by binding to FVIIa and allosterically activating the enzyme. An alanine-scanning mutagenesis study of the region surrounding these residues in the linear sequence of TF (residues 157-167) also showed that this region of TF was important for macromolecular substrate recognition. Ruf, et al., *J Biol Chem* 267:22206-22210 (1992b). Recently, it has been reported that these same two residues on TF play a role in the rate of binding of complexes of FXa and tissue factor pathway inhibitor to the TF-FVIIa complex. Rao, L.V.M. and Ruf, W., *Biochemistry* 34:10867-10871 (1995).

Subsequent to the mutagenesis studies cited above, the x-ray crystal structure of the isolated extracellular region of TF was reported. Harlos, et al., *Nature* 370:662-666 (1994); Muller, et al., *Biochemistry* 33:10864-10870 (1994). The protein is a member of the cytokine/growth factor receptor family and is composed of two fibronectin type-III modules joined together at an angle of roughly 125°. The putative FVIIa binding site on TF identified by alanine-scanning mutagenesis comprises side chains located on both modules and the interface region joining the two. Martin, et al., *FASEB J* 9:852-859 (1995). Residues K¹⁶⁵ and K¹⁶⁶ are exposed to solvent and located near the end of module 2, close to the expected phospholipid surface. Interestingly, these residues lie on the opposite side of TF relative to the putative FVIIa binding region. This assignment was confirmed in a recent preliminary report of the x-ray crystal structure of the complex of FVIIa and the extracellular domain of TF. Banner, et al., *Thromb Haemost* 73:1183 (1995).

Based on their location in the crystal structure of TF, we hypothesized that K¹⁶⁵ and K¹⁶⁶ are positioned to interact directly with the Gla-domain of FX, and that this interaction is important for efficient use of FX as a substrate by the TF-FVIIa complex. In the present study we report evidence that TF residues K¹⁶⁵ and K¹⁶⁶ contribute to activation of intact but not Gla-domainless FX. Furthermore, the interaction between the Gla-domain of FX and this region of TF may be facilitated by the Gla-domain of FVIIa.

The following materials are employed in the examples given below: Chromozym t-PA (N-methylsulfonyl-D-phenylalanyl-glycyl-arginyl-4-nitroanilide acetate) was from Boehringer Mannheim; Spectrozyme FXa (MeO-CO-D-cyclohexylglycyl-glycyl-arginyl-4-nitroanilide acetate) was from American Diagnostica (Greenwich, CF); and S-2222 (N-benzoyl-isoleucyl-glutamyl-glycyl-arginyl4-nitroanflide hydrochloride) was from Chromogenix. Poly(L-lysine), Tris base, Lubrol-PX EDTA, Antifoam C, and Tricine (N-tris-(hydroxymethyl)methylglycine) were from Sigma. BSA (bovine serum albumin, fraction V, fatty acid-free), Mes (2(N-morpholino)- ethanesulfonic acid), Hepes, and octyl-o-D-glucopyranoside were from Calbiochem. PC and PS were from Avanti Polar Lipids (Pelham, AL). Non-sterile and untreated 96-weR microplates were from Coming (Corning, NY).

### Example 1

Protein Purification. Recombinant human TF (wild-type and mutant) was expressed in *E. coli* and purified as originally described for soluble TF (Rezaie, et al., *Protein Express Purif* 3:453-460 (1992)) with modifications for the production of membrane-anchored TF as previously described (Neuenschwander, et al., *Biochemistry* 34:13988-13993(1995)). The form of membrane-anchored TF used in this study has had most of the cytoplasmic domain removed, and consists of amino acids 1 through 244 according to the numbering system used previously. Morrissey, et al., *Cell* 50:129-135 (1987). The cytoplasmic domain of TF is dispensable for activity and is commonly omitted from recombinant TF since it interferes with production in, and purification from, *E. coli.* Paborsky, et al., *Biochemistry* 28:8072-8077 (1989). The purified mutant TF proteins migrated as a single band with Mᵣ = 30,000 as did wild type TF when subjected to polyacrylamide gel electrophoresis (data not shown). Grossly correct folding of wild-type and mutant TF proteins was verified by examining the support of FVIIa amidolytic activity as described (Neuenschwander, et al., *Thromb Haemost* 70:970-977 (1993)), and the binding affinity for FVIIa as described. Neuenschwander, P.F. and Morrissey J.H., *J Biol Chem* 269:8007-8013 (1994) and correction in *J Biol Chem* 269:16983. TF was relipidated by the octyl-β-D-glucopyranoside dialysis method (Mimms, et al., *Biochemistry* 20:833-840 (1981)) as originally applied to TF. Bach, et al., *Biochemistry* 25:4007-4020(1986). Typically, TF was incorporated into phospholipid vesicles prepared from PC or PCPS, at a molar ratio of TF to total phospholipid of about 1:8000. Blank phospholipid vesicles were prepared in the same manner but in the absence of TF. The amount of available TF on the outside of the vesicles was quantified as described. Neuenschwander, P.F. and Morrissey, J.H., *J Biol Chem* 269:8007-8013 (1994) and correction in *J Biol Chem* 269:16983 (1994). Other proteins were purified as described: FX from human plasma (Le Bonniec, et al., *J Biol Chem* 267:6970-6976 (1992)), FVIIa from human plasma (Neuenschwander, P.F. and Morrissey, J.H., *J Biol* Chem 267:14477-14482 (1992)), recombinant Gla-domainless FX ("GDFX") (Rezaie, et al., *J Biol Chem* 268:8176-8180 (1993)), and FIX from human plasma (Thompson, A.R., *J Clin Invest* 59:900-910 (1977)).

*Mutagenesis of TF.* The above-referenced construct encoding membrane-anchored TF (Neuenschwander, et al., *Biochemistry* 34:13988-13993(1995)) was used for the production of TF mutants. Residues K¹⁶⁵ and K¹⁶⁶ were mutated via polymerase chain reaction (PCR)-based mutagenesis (Horton, et al., *BioTechniques* 8:528-535 (1990)) using appropriate flanking primers and the following pairs of mutagenic primers (mutations underlined): 5'-TCA AGT TCA GGA GCG GCG ACA GCC AAA ACA-3' (SEQ ID NO:43) and 5'-CGC CGC TCC TGA ACT TGA AGA TTT CC-3' (SEQ ID NO:44) (A¹⁶⁵A¹⁶⁶ mutant); 5'-TCA AGT TCA GGA GAA GAA ACA GCC AAA ACA-3' (SEQ ID NO:45) and 5'-GT TTC TTC TCC TGA ACT TGA AGA TTT CC-3' (SEQ ID NO:46) (E¹⁶⁵E¹⁶⁶ mutant); and 5'-TCA AGT TCA GGA CAG CAG ACA GCC AAA ACA-3' (SEQ ID NO:47) and 5'-T CTG CTG TCC TGA ACT TGA AGA TTT CC-3' (SEQ ID NO:48) (Q ¹⁶⁵Q¹⁶⁶ mutant). PCRs were hot-started with Ampliwax (Perkin-Elmer Cetus) and amplified for 25 cycles using 30 sec at 95°C, 1 min at 50°C, and 30, sec at 72°C per cycle. The coding sequences of all TF cDNA constructs were verified by DNA sequencing.

### Example 2

*Activity assays.* Activation of FX by the various TF-FVIIa complexes was quantified using a two-stage discontinuous chromogenic assay essentially as previously described (Fiore, et al., *Blood* 80:3127-3134 (1992)), but with some modification. Briefly, in the first stage, TF in phospholipid vesicles was incubated with FVIIa in HBSA (20 mM Hepes-NaOH pH 7.5, 100 mM NaCl, 5 mM CaCl₂ 0.1% bovine serum albumin) for 5 minutes at 37°. Prewarmed substrate solution (FX) was then added to the mixture to initiate the reaction, after which 20-µl samples were removed at varying times into 25 µl of stop buffer (20 mM Mes-NaOH pH 5.8,20 mM EDTA, 8% Lubrol-PX, 0.01% Antifoam C) and maintained on ice. In the second stage, the pH of the quenched samples was adjusted to 8.3 by a 10-µl addition of 2 M Tricine pH 8.4, and the FXa present in each sample was subsequently determined at ambient temperature by adding 50 µl of Spectrozyme FXa to yield a final substrate concentration of 0.48 mM. The change in absorbance at 405 nm was monitored over 10 min using a Vmax Microplate Reader (Molecular Devices Corp, Menlo Park, CA) and initial rates were determined (mOD min-¹). These values were then converted to nM FXa by comparison to a standard line prepared with purified human FXa. For assays in solution, rates of FX activation were measured as above, except that TF was not reconstituted into phospholipid vesicles, and all solutions contained 0.1% Triton.

Activation of FIX by the TF-FVIIa complex was assayed as described (Fiore, et al., *Blood* 80:3127-3134 (1992)) with the following modifications. In the first stage, 1 nM TF relipidated in 10-14 µM PCPS was incubated with 50 pM FVIIa in HBSA at 37°C for 5 min, after which FIX was added to a final concentration of 150 nM. Timed samples (10 µl) were added to 40 µl of stop buffer (0.5 mM Tris-HCl, pH 7.0, 1 mM EDTA, 0.2% Lubrol PX, 0.1% bovine serum albumin, 0.01% Antifoam C) and maintained on ice. In the second stage, 10 µl of each quenched sample was added to a separate microplate well containing 40 µl FX (150 nM) in 20 mM Hepes-NaOH, pH 7.5, 57.5 mM NaCl, 5 mM CaCl₂, 0.1% bovine serum albumin. FXa generation and hydrolysis of chromogenic substrate by FXa were started simultaneously by adding 50 µl of a mixture of S-2222 and poly(L-lysine) to final concentrations of 0.5 mM and 10 nM, respectively. The absorbance was monitored over 40 min at both 405 nm and 490 nm to correct for scatter due to the high level of additives in the reaction. The resulting absorbance-difference profile was then used to calculate initial rates of activated factor IX ("FIXa") generation as previously described (Fiore, et al., *Blood* 80:3127-3134 (1992)). Control experiments indicated that FVIIa carried over from the first stage had negligible activity in the second stage of the assay.

### Example 3

*Coagulation assays*. The ability of mutant or wild type TF to support the clotting of plasma was measured using a model ST4 coagulometer (American Bioproducts Co., Parsippany, NJ). TF solutions were prepared using varying concentrations of either wild type or mutant TF in phospholipid vesicles (typically, 20% phosphatidyl serine and 80% phosphatidyl choline) dissolved in HBSA (20 mM Hepes sodium hydroxide, pH 7.5, 100 mM sodium chloride, 5 mM calcium chloride, and 0.1% bovine serum albumin). It has been found that combinations of phosphatidyl serine, phosphatidyl choline and/or phosphatidyl ethanolamine are particularly desirable. Citrated plasma mixtures were prepared by mixing together varying proportions of normal human plasma and specific factor-deficient plasmas. The various factor-deficient plasmas were from congenitally factor-deficient individuals, as purchased from George King Bio-Medical, Inc. (Overland Park, KS). These deficient plasmas were certified by the supplier to contain less than 1% the normal level of the indicated factor. Calcium chloride solution consisted of 25 mM CaCl₂ dissolved in water. The assays were performed by mixing 50 µl of TF solution (wild type or mutant) with 50 µl of the desired citrated plasma mixture in a cuvette in the ST4 coagulometer. After the mixtures were warmed to 37°C, 50 µl of pre-warmed calcium chloride solution was added and the time to clot formation was recorded.

### RESULTS

Amidolytic activity and FVIIa binding. It has previously been reported that mutation of two adjacent K residues to A at positions 165 and 166 of TF greatly decreased the ability of the resulting TF-FVIIa complexes to activate FX. Roy, et al., *J Biol Chem* 266:22063-22066 (1991). However, these mutations did not decrease the affinity for FVIIa and the mutants showed normal enhancement of FVIIa amidolytic activity. In the present study we chose to mutate these two residues to A or E in order to examine the effects of truncating these side chains or reversing their charge. In addition, Q substitutions were included as an approximately isosteric control for the E mutations without charge reversal. Because previous studies indicated that the effects of mutating K¹⁶⁵ and K¹⁶⁶ separately were additive in the double mutant, we chose only to explore double mutants at positions 165 and 166 in the present study. As shown in Table I, all three TF mutants exhibited wild-type levels of cofactor function toward the amidolytic activity of FVIIa. The mutants also exhibited affinities for FVIIa that were at least as strong as the affinity of wild-type TF for FVIIa (Table I).

*FX and FIX activation by TF-FVIIa*. As shown in Fig. 6, mutating K¹⁶⁵ K¹⁶⁶ to A¹⁶⁵ A¹⁶⁶ severely impaired FX activation by the TF-FVIIa complex. Furthermore, the charge-reversal mutations (E¹⁶⁵E¹⁶⁶) impaired FX activation more severely while the relatively conservative mutations (Q¹⁶⁵Q¹⁶⁶) impaired FX activation less severely. The impairments in FX activation were not dependent on PS in the vesicles since the mutants showed even greater defects in FX activation when TF was relipidated in pure PC vesicles. As with FX activation, mutating K¹⁶⁵K¹⁶⁶ to A¹⁶⁵ A¹⁶⁶ impaired FIX activation (Fig. 7). The charge-reversal mutations (E¹⁶⁵E¹⁶⁶) also impaired FIX activation more severely, while the more-conservative mutations (Q¹⁶⁵Q¹⁶⁶) impaired FIX activation less severely. However, the relative defect of these TF mutants for supporting FIX activation (14-26% of wild-type) was significantly smaller in magnitude than for supporting FX activation (3.9-17% of wild-type). Surprisingly therefore, the TF mutants containing substitutions at positions 165 and 166 retained significantly more activity in supporting the activation of factor IX than they did in supporting the activation of factor X. In addition, the substitution of glutamine for

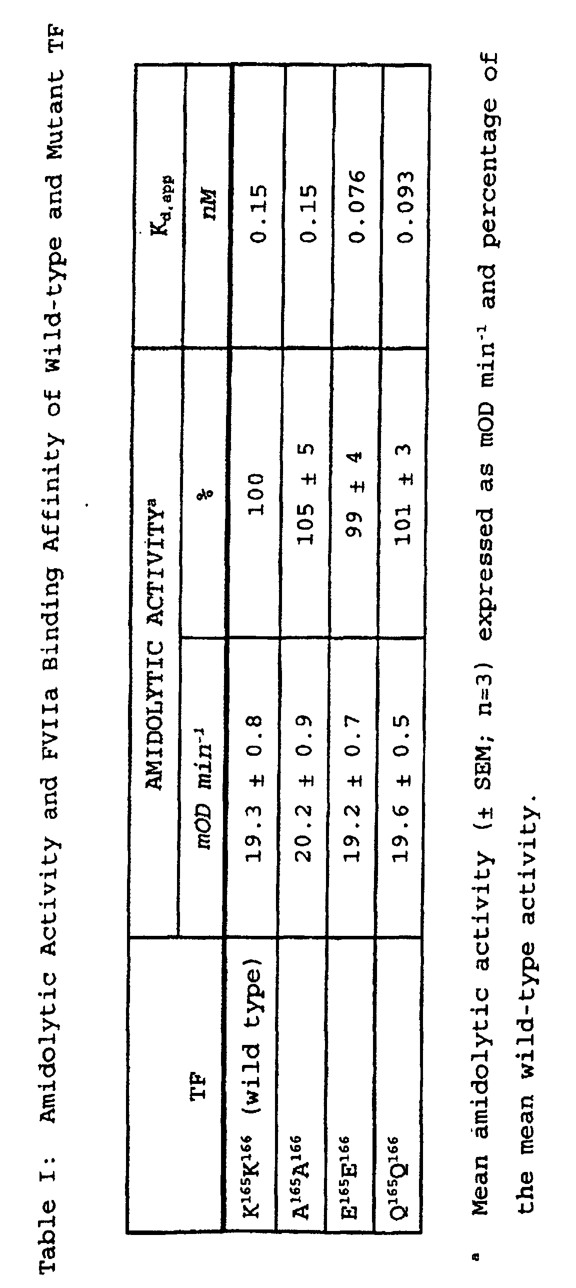

the lysines at positions 165 and 166, to yield TF Q¹⁶⁵Q¹⁶⁶, resulted in a TF mutant that retained sufficient activity that it could potentially be used in clot-based assays of human plasma.

Mutation of K¹⁶⁵K¹⁶⁶ in TF to A¹⁶⁵ A¹⁶⁶ substantially reduced the ability of the resulting TF-FVIIa complexes to activate FX while not affecting the affinity of TF for FVIIa or the enhancement of FVIIa amidolytic activity. This result confirms those of previous reports. Roy, et al., *J Biol Chem* 266:22063-22066 (1991); Ruf, et al., *J Biol Chem* 267:6375-6381 (1992a); Ruf, et al., *J Biol Chem* 267:22206-22210 (1992b). In addition, these results were extended to show that charge-reversal mutations of these residues (E¹⁶⁵E¹⁶⁶) caused an even greater defect in FX activation, while more conservative substitution (Q¹⁶⁵Q¹⁶⁶) supported somewhat higher levels of FX activation than observed with the A¹⁶⁵A¹⁶⁶ mutant. As with the A¹⁶⁵A¹⁶⁶ mutant, the E¹⁶⁵E¹⁶⁶ and Q¹⁶⁵Q¹⁶⁶ mutants had normal affinity for FVIIa and showed normal enhancement of FVIIa amidolytic activity. The results also support the idea of a general defect in macromolecular substrate recognition when these residues are mutated, since FIX activation was also severely impaired by mutating K¹⁶⁵ and K¹⁶⁶. Surprisingly, the defect in FIX activation exhibited by these mutants was notably less severe than that observed for FX activation. Therefore, residues K¹⁶⁵ and K¹⁶⁶ of TF appear to be more important in enhancing FX activation than FIX activation.

*Clotting times with factor-deficient plasmas.* The experiments present above demonstrated that, surprisingly, the TF mutants containing amino acid substitutions at positions 165 and 166 had less severe defects in the activation of factor IX than they did in the activation of factor X. These results indicated that these TF mutants would exhibit a substrate preference in favor of factor IX in whole plasma. This is in contrast to wild type TF, which exhibits a marked substrate preference for factor X in whole plasma. The most preferable mutant was found to result from the substitution of the lysine residues at positions 165 and 166 by glutamine which resulted in a protein (TF Q¹⁶⁵Q¹⁶⁶), which exhibits the best overall activity and therefore allows the clotting time to be shorter, which is generally desirable. TF A¹⁶⁵A¹⁶⁶ and TF E¹⁶⁵E¹⁶⁶ exhibited suitable activity as well, although longer clotting times were observed than when TF Q¹⁶⁵Q¹⁶⁶ was used. The sensitivity of TF Q¹⁶⁵Q¹⁶⁶ to various clotting factors in a clot-based assay was tested as indicated in Fig. 1 through Fig. 5. In these experiments, the concentrations of wild type or mutant TF were adjusted to give clotting times with citrated, normal human plasma of approximately 25 to 30 seconds. Fig. 1 indicates that, compared to wild type TF, TF Q¹⁶⁵Q¹⁶⁶ has greatly enhanced sensitivity to FIX in plasma. This can be seen by the approximately equivalent clotting times at 0% FIX deficient plasma (i.e., 100% normal plasma), but the greatly prolonged clotting times observed with TF Q¹⁶⁵Q¹⁶⁶ as the percentage of FIX deficient plasma is increased. Thus, the clotting time for wild type TF was prolonged by an insignificant amount in 100% FIX-deficient plasma, while the clotting time for TF Q¹⁶⁵Q¹⁶⁶ was prolonged significantly in 100% FIX-deficient plasma. Similar differences are readily apparent in the prolongation of the clotting times at 50%, 75%, and 90% FIX-deficient plasma.

Fig. 2 indicates that, compared to wild type TF, TF Q¹⁶⁵Q¹⁶⁶ has greatly enhanced sensitivity to FVIII in plasma. This can be seen by the equivalent clotting times at 0% FVIII deficient plasma (i.e., 100% normal plasma), but the greatly prolonged clotting times observed with TF Q¹⁶⁵Q¹⁶⁶ as the percentage of FVIII deficient plasma is increased. Thus, the clotting time for wild type TF was only very slightly prolonged in 100% FVIII-deficient plasma, while the clotting time for TF Q¹⁶⁵Q¹⁶⁶ was prolonged significantly in 100% FVIII-deficient plasma. Similar differences are readily apparent in the prolongation of the clotting times at 50%, 75%, and 90% FVIII-deficient plasma. Fig. 3 indicates that wild type TF and TF Q¹⁶⁵Q¹⁶⁶ have essentially equivalent sensitivities to FX in plasma. Fig. 4 indicates that TF Q¹⁶⁵Q¹⁶⁶ is somewhat more sensitive to FVII levels in plasma than is wild type TF. Fig. 5 indicates that wild type TF and TF Q¹⁶⁵Q¹⁶⁶ have essentially equivalent sensitivities to FV in plasma.

In summary, mutant TF containing amino acid substitutions at positions 165 and 166 exhibited substantial sensitivity in their clotting activity toward FVIII and FIX. As expected, wild type TF was essentially insensitive to the levels of FVIII or FIX in plasma. This means that individuals deficient in FVIII or FIX have prolonged clotting times when clotting is initiated with TF Q¹⁶⁵Q¹⁶⁶, but normal clotting times when clotting is initiated with wild type TF.

In summary, mutation of TF in positions 165 and 166 provides for a reagent useful in measuring factors VIII and IX.

## Claims

1. A method for measuring the concentration of clotting factor VIII or IX in a patient's plasma sample, comprising the steps of:
(a) mixing a quantity of a plasma sample with a mutated tissue factor reagent, said mutated tissue factor reagent comprising an amino acid sequence mutated at position 165 and/or 166 as numbered according to SEQ ID NO:1 and capable of binding to factor VIIa and promoting factor VIIa amidolytic activity but deficient in supporting factor IX and factor X activation, said to form a first mixture ;
(b) adding an effective amount of calcium ions to said first mixture to initiate clotting ;
(c) determining a plasma sample clotting time measured from a time of addition of said calcium ions to a time of clot formation ; and
(d) comparing said plasma sample clotting time to a standard clotting time determined for said clotting factor of a known concentration.

2. The method of Claim 1, wherein said tissue factor sequence comprises at least a portion of wild-type tissue factor of SEQ ID NO:1, starting from about amino acid -5 to about 5 and ending at about 258 to about 263.

3. The method of Claim 1, wherein said tissue factor sequence comprises at least a portion of SEQ ID NO:1, starting from about amino acid -5 to about 5 and ending at about 214 to about 248.

4. The method of Claim 2, wherein said sequence is substituted with a substitute amino acid at at least one position, wherein said position is 154, 159, 163, 164, 178, 180, 201 or 204.

5. The method of Claim 3, wherein said sequence is substituted with a substitute amino acid at at least one position, wherein said position is 154, 159, 163, 164, 165, 166, 178, 180, 201 or 204.

6. The method of Claim 4, wherein said substitute amino acid is selected from de group consisting of glutamine, alanine and glutamic acid and combinations thereof.

7. The method of Claim 5, wherein said substitute amino acid is selected from the group consisting of glutamine, alanine and glutamic acid and combinations thereof.

8. The method of Claim 2, wherein said sequence is substituted at position 165.

9. The method of Claim 3, wherein said sequence is substituted at position 165.

10. The method of Claim 2, wherein said sequence is substituted at position 166.

11. The method of Claim 3, wherein said sequence is substituted at position 166.

12. The method of Claim 2, wherein said sequence is substituted at positions 165 and 166.

13. The method of Claim 3, wherein said sequence is substituted at positions 165 and 166.

14. The method of Claims 8, 9, 10, 11, 12 or 13, wherein said sequence is substituted with a substitute amino acid selected from the group consisting of glutamine, alanine and glutamic acid and combinations thereof.

15. The method of Claim 2, wherein said tissue factor sequence is SEQ ID NO:3.

16. The method of Claim 2, wherein said tissue factor sequence is SEQ ID NO:6.

17. The method of Claim 2, wherein said tissue factor sequence is SEQ ID NO:9.

18. The method of Claim 2, wherein said tissue factor sequence is SEQ ID NO:4.

19. The method of Claim 2, wherein said tissue factor sequence is SEQ ID NO:7.

20. The method of Claim 2, wherein said tissue factor sequence is SEQ ID NO:14.

21. The method of Claim 2, wherein said tissue factor sequence is SEQ ID NO:5.

22. The method of Claim 2, wherein said tissue factor sequence is SEQ ID NO:8.

23. The method of Claim 2, wherein said tissue factor sequence is SEQ ID NO:17.

24. The method of Claim 3, wherein said tissue factor is SEQ ID NO:20.

25. The method of Claim 3, wherein said tissue factor sequence is SEQ ID NO:23.

26. The method of Claim 3, wherein said tissue factor sequence is SEQ ID NO:26.

27. The method of Claim 3, wherein said tissue factor sequence is SEQ ID NO:21.

28. The method of Claim 3, wherein said tissue factor sequence is SEQ ID NO:24.

29. The method of Claim 3, wherein said tissue factor sequence is SEQ ID NO:31.

30. The method of Claim 3, wherein said tissue factor sequence is SEQ ID NO:22.

31. The method of Claim 3, wherein said tissue factor sequence is SEQ ID NO:25.

32. The method of Claim 3, wherein said tissue factor sequence is SEQ ID NO:34.

33. The method of Claim 1, 2 or 3, wherein said mixing further comprises mixing a phospholipid composition with said plasma sample.

34. The method of Claim 33, wherein said phospholipid comprises a combination of phosphatidyl choline and phosphatidyl serine.

35. The method of Claim 33, wherein said phospholipid comprises phosphatidyl choline, phosphatidyl serine and phosphatidyl ethanolamine.

36. The method of Claim 33, wherein said phospholipid composition is added with said reagent to said sample.

37. The method of Claim 1, wherein said calcium ions are added simultaneously with said reagent to said sample.

38. The method of Claim 1, wherein said calcium ions are added subsequent to said reagent to said sample.

39. Use of a mutated tissue factor reagent in a clotting assay for directly measuring the concentration of factor VIII or factor IX, wherein said mutated tissue factor reagent comprises an amino acid sequence mutated at position 165 and/or 166 as numbered according to SEQ ID NO:1 and capable of binding to factor VIIa and promoting factor VIIa amidolytic activity but deficient in supporting factor IX and factor X activation.

40. Use according to claim 39, wherein said mutated tissue factor reagent is SEQ ID NO:35.

41. Use according to claim 40, wherein Xaa in SEQ ID NO:35 is selected from the group consisting of glutamine, alanine, and glutamic acid.

42. Use according to claim 39, wherein said mutated tissue factor reagent is SEQ ID NO:36.

43. Use according to claim 42, wherein Xaa in SEQ ID NO:36 is selected from the group consisting of glutamine, alanine, and glutamic acid.

44. Use according to claim 39, wherein said mutated tissue factor reagent is SEQ ID NO:37.

45. Use according to claim 44, wherein Xaa in SEQ ID NO:3 is selected from the group consisting of glutamine, alanine, and glutamic acid and combinations thereof.

46. Use according to claim 39, wherein said mutated tissue factor reagent is SEQ ID NO:38.

47. Use according to claim 46, wherein Xaa in SEQ ID NO:38 is selected from the group consisting of glutamine, alanine, and glutamic acid.

48. Use according to claim 39, wherein said mutated tissue factor reagent is SEQ ID NO:39.

49. Use according to claim 48, wherein Xaa in SEQ ID NO:38 is selected from the group consisting of glutamine, alanine, and glutamic acid.

50. Use according to claim 39, wherein said mutated tissue factor reagent is SEQ ID NO:40.

51. Use according to claim 49, wherein Xaa in SEQ ID NO:40 is selected from the group consisting of glutamine, alanine, and glutamic acid.

## Patentansprüche

1. Verfahren zur Messung der Konzentration von Gerinnungsfaktor VIII oder IX in einer Plasmaprobe eines Patienten, umfassend die Schritte:
a) Mischen einer Menge einer Plasmaprobe mit einem Mutanten-Gewebefaktor-Reagenz, wobei das Mutanten-Gewebefaktor-Reagenz eine Aminosäuresequenz umfaßt, die an Position 165 und/oder 166, wie gemäß SEQ-ID-Nr. 1 gezählt, mutiert ist und in der Lage ist, an Faktor VIIa zu binden und die amidolytische Aktivität von Faktor VIIa zu fördern, jedoch nicht in der Lage ist, die Aktivierung von Faktor IX und Faktor X zu unterstützen, um eine erste Mischung zu bilden;
b) Zugeben einer wirksamen Menge an Calciumionen zu der ersten Mischung, um die Gerinnung einzuleiten;
c) Bestimmen einer Gerinnungszeit der Plasmaprobe, gemessen vom Zeitpunkt der Zugabe der Calciumionen bis zum Zeitpunkt der Gerinnselbildung; und
d) Vergleichen der Gerinnungszeit der Plasmaprobe mit einer Standard-Gerinnungszeit, die für diesen Gerinnungsfaktor bei einer bekannten Konzentration bestimmt wurde.

2. Verfahren nach Anspruch 1, wobei die Gewebefaktor-Sequenz mindestens einen Teil des Wildtyp-Gewebefaktors von SEQ-ID-Nr. 1 umfaßt, der bei etwa Aminosäure -5 bis etwa 5 anfängt und bei etwa 258 bis etwa 263 endet.

3. Verfahren nach Anspruch 1, wobei die Gewebefaktor-Sequenz mindestens einen Teil von SEQ-ID-Nr. 1 umfaßt, der bei etwa Aminosäure -5 bis etwa 5 anfängt und bei etwa 214 bis etwa 248 endet.

4. Verfahren nach Anspruch 2, wobei die Sequenz mit einer Ersatzaminosäure an mindestens einer Position substituiert ist, wobei diese Position 154, 159, 163, 164, 178, 180, 201 oder 204 ist.

5. Verfahren nach Anspruch 3, wobei die Sequenz mit einer Ersatzaminosäure an mindestens einer Position substituiert ist, wobei diese Position 154, 159, 163, 164, 165, 166, 178, 180, 201 oder 204 ist.

6. Verfahren nach Anspruch 4, wobei die Ersatzaminosäure aus der Gruppe ausgewählt ist, die aus Glutamin, Alanin und Glutaminsäure und Kombinationen davon besteht.

7. Verfahren nach Anspruch 5, wobei die Ersatzaminosäure aus der Gruppe ausgewählt ist, die aus Glutamin, Alanin und Glutaminsäure und Kombinationen davon besteht.

8. Verfahren nach Anspruch 2, wobei die Sequenz an Position 165 substituiert ist.

9. Verfahren nach Anspruch 3, wobei die Sequenz an Position 165 substituiert ist.

10. Verfahren nach Anspruch 2, wobei die Sequenz an Position 166 substituiert ist.

11. Verfahren nach Anspruch 3, wobei die Sequenz an Position 166 substituiert ist.

12. Verfahren nach Anspruch 2, wobei die Sequenz an den Positionen 165 und 166 substituiert ist.

13. Verfahren nach Anspruch 3, wobei die Sequenz an den Positionen 165 und 166 substituiert ist.

14. Verfahren nach einem der Ansprüche 8, 9, 10, 11, 12 oder 13, wobei die Sequenz mit einer Ersatzaminosäure substituiert ist, die aus der Gruppe ausgewählt ist, welche aus Glutamin, Alanin und Glutaminsäure und Kombinationen davon besteht.

15. Verfahren nach Anspruch 2, wobei die Gewebefaktor-Sequenz SEQ-ID-Nr. 3 ist.

16. Verfahren nach Anspruch 2, wobei die Gewebefaktor-Sequenz SEQ-ID-Nr. 6 ist.

17. Verfahren nach Anspruch 2, wobei die Gewebefaktor-Sequenz SEQ-ID-Nr. 9 ist.

18. Verfahren nach Anspruch 2, wobei die Gewebefaktor-Sequenz SEQ-ID-Nr. 4 ist.

19. Verfahren nach Anspruch 2, wobei die Gewebefaktor-Sequenz SEQ-ID-Nr. 7 ist.

20. Verfahren nach Anspruch 2, wobei die Gewebefaktor-Sequenz SEQ-ID-Nr. 14 ist.

21. Verfahren nach Anspruch 2, wobei die Gewebefaktor-Sequenz SEQ-ID-Nr. 5 ist.

22. Verfahren nach Anspruch 2, wobei die Gewebefaktor-Sequenz SEQ-ID-Nr. 8 ist.

23. Verfahren nach Anspruch 2, wobei die Gewebefaktor-Sequenz SEQ-ID-Nr. 17 ist.

24. Verfahren nach Anspruch 3, wobei die Gewebefaktor-Sequenz SEQ-ID-Nr. 20 ist.

25. Verfahren nach Anspruch 3, wobei die Gewebefaktor-Sequenz SEQ-ID-Nr. 23 ist.

26. Verfahren nach Anspruch 3, wobei die Gewebefaktor-Sequenz SEQ-ID-Nr. 26 ist.

27. Verfahren nach Anspruch 3, wobei die Gewebefaktor-Sequenz SEQ-ID-Nr. 21 ist.

28. Verfahren nach Anspruch 3, wobei die Gewebefaktor-Sequenz SEQ-ID-Nr. 24 ist.

29. Verfahren nach Anspruch 3, wobei die Gewebefaktor-Sequenz SEQ-ID-Nr. 31 ist.

30. Verfahren nach Anspruch 3, wobei die Gewebefaktor-Sequenz SEQ-ID-Nr. 22 ist.

31. Verfahren nach Anspruch 3, wobei die Gewebefaktor-Sequenz SEQ-ID-Nr. 25 ist.

32. Verfahren nach Anspruch 3, wobei die Gewebefaktor-Sequenz SEQ-ID-Nr. 34 ist.

33. Verfahren nach Anspruch 1, 2 oder 3, wobei das Mischen ferner das Mischen einer Phospholipid-Zusammensetzung mit der Plasmaprobe umfaßt.

34. Verfahren nach Anspruch 33, wobei das Phospholipid eine Kombination von Phosphatidylcholin und Phosphatidylserin umfaßt.

35. Verfahren nach Anspruch 33, wobei das Phospholipid Phosphatidylcholin, Phosphatidylserin und Phosphatidylethanolamin umfaßt.

36. Verfahren nach Anspruch 33, wobei die Phospholipid-Zusammensetzung mit dem Reagenz der Probe zugegeben wird.

37. Verfahren nach Anspruch 1, wobei die Calciumionen gleichzeitig mit dem Reagenz der Probe zugegeben werden.

38. Verfahren nach Anspruch 1, wobei die Calciumionen nach dem Reagenz der Probe zugegeben werden.

39. Verwendung eines Mutanten-Gewebefaktor-Reagenzes in einem Gerinnungsassay zur direkten Messung der Konzentration von Faktor VIII oder Faktor IX, wobei das Mutanten-Gewebefaktor-Reagenz eine Aminosäuresequenz umfaßt, die an Position 165 und/oder 166, wie gemäß SEQ-ID-Nr. 1 gezählt, mutiert ist und imstande ist, an Faktor Vlla zu binden und die amidolytische Aktivität von Faktor VIIa zu fördern, jedoch nicht in der Lage ist, die Aktivierung von Faktor IX und Faktor X zu unterstützen.

40. Verwendung nach Anspruch 39, wobei das Mutanten-Gewebefaktor-Reagenz SEQ-ID-Nr. 35 ist.

41. Verwendung nach Anspruch 40, wobei Xaa in SEQ-ID-Nr. 35 aus der Gruppe ausgewählt ist, die aus Glutamin, Alanin und Glutaminsäure besteht.

42. Verwendung nach Anspruch 39, wobei das Mutanten-Gewebefaktor-Reagenz SEQ-ID-Nr. 36 ist.

43. Verwendung nach Anspruch 42, wobei Xaa in SEQ-ID-Nr. 36 aus der Gruppe ausgewählt ist, die aus Glutamin, Alanin und Glutaminsäure besteht

44. Verwendung nach Anspruch 39, wobei das Mutanten-Gewebefaktor-Reagenz SEQ-ID-Nr. 37 ist.

45. Verwendung nach Anspruch 44, wobei Xaa in SEQ-ID-Nr. 37 aus der Gruppe ausgewählt ist, die aus Glutamin, Alanin und Glutaminsäure und Kombinationen davon ausgewählt ist.

46. Verwendung nach Anspruch 39, wobei das Mutanten-Gewebefaktor-Reagenz SEQ-ID-Nr. 38 ist.

47. Verwendung nach Anspruch 46, wobei Xaa in SEQ-ID-Nr. 38 aus der Gruppe ausgewählt ist, die aus Glutamin, Alanin und Glutaminsäure besteht.

48. Verwendung nach Anspruch 39, wobei das Mutanten-Gewebefaktor-Reagenz SEQ-ID-Nr. 39 ist.

49. Verwendung nach Anspruch 48, wobei Xaa in SEQ-ID-Nr. 39 aus der Gruppe ausgewählt ist, die aus Glutamin, Alanin und Glutaminsäure besteht.

50. Verwendung nach Anspruch 39, wobei das Mutanten-Gewebefaktor-Reagenz SEQ-ID-Nr. 40 ist.

51. Verwendung nach Anspruch 49, wobei Xaa in SEQ-ID-Nr. 40 aus der Gruppe ausgewählt ist, die aus Glutamin, Alanin und Glutaminsäure besteht

## Revendications

1. Procédé de mesure de la concentration de facteur VIII ou IX dans un échantillon plasmatique d'un malade, comprenant les étapes consistant en :
(a) le mélange d'une quantité d'un échantillon plasmatique avec un réactif à base de facteur tissulaire produit par mutagenèse, ledit réactif à base de facteur tissulaire produit par mutagenèse comprenant une séquence d'acides aminés ayant fait l'objet de mutation en position, 165 et/ou 166 selon la numérotation de SEQ ID NO : 1 et capable de se lier au facteur VIIa et de promouvoir l'activité amidolytique du facteur VIIa sans être en mesure d'entretenir ou médier l'activation du facteur IX et du facteur X, pour former un premier mélange ;
(b) l'addition d'une quantité efficace d'ions calcium audit premier mélange pour initier la coagulation ;
(c) la détermination du temps de coagulation plasmatique mesuré à partir de l'instant d'addition desdits ions calcium jusqu'à la formation du caillot ; et
(d) la comparaison dudit temps de coagulation de l'échantillon plasmatique avec un temps de coagulation étalon déterminé pour ledit facteur de coagulation de concentration connue.

2. Procédé selon la revendication 1, dans lequel ladite séquence du facteur tissulaire comprend au moins un fragment du facteur tissulaire de type sauvage correspondant à SEQ ID NO : 1, commençant par l'acide aminé en position -5 à 5 environ et se terminant par l'acide aminé en position 258 à 263 environ.

3. Procédé selon la revendication 1, dans lequel ladite séquence de facteur tissulaire comprend au moins une partie de SEQ ID NO : 1, commençant par l'acide aminé en position -5 à 5 environ et se terminant par l'acide aminé en position 214 à 248 environ.

4. Procédé selon la revendication 2, dans lequel ladite séquence est substituée par un acide aminé de substitution à au moins une position, dans lequel ladite position est 154, 159, 163, 164, 178, 180, 201 ou 204.

5. Procédé selon la revendication 3, dans lequel ladite séquence est substituée par un acide aminé de substitution à au moins une position, dans lequel ladite position est 154, 159, 163, 165, 166, 178, 180, 201 ou 204.

6. Procédé selon la revendication 4, dans lequel ledit acide aminé de substitution est choisi dans le groupe consistant en la glutamine, l'alanine et l'acide glutamique et les combinaisons de ceux-ci.

7. Procédé selon la revendication 5, dans lequel ledit acide aminé de substitution est choisi dans le groupe consistant en la glutamine, l'alanine et l'acide gitamique et les combinaison de ceux-ci.

8. Procédé selon la revendication 2, dans lequel ladite séquence est substituée en position 165.

9. Procédé selon la revendication 3, dans lequel ladite séquence est substituée en position 165.

10. Procédé selon la revendication 2, dans lequel ladite séquence est substituée en position 166.

11. Procédé selon la revendication 3, dans lequel ladite séquence est substituée en position 166.

12. Procédé selon la revendication 2, dans lequel ladite séquence est substituée en positions 165 et 166.

13. Procédé selon la revendication 3, dans lequel ladite séquence est substituée en positions 165 et 166.

14. Procédé selon la revendication 8, 9, 10, 11, 12 ou 13, dans lequel ladite séquence est substituée par un acide aminé de substitution choisi dans le groupe consistant en la glutamine, l'alanine et l'acide glutamique et les combinaisons de ceux-ci.

15. Procédé selon la revendication 2, dans lequel ladite séquence de facteur tissulaire correspond à SEQ ID NO : 3.

16. Procédé selon la revendication 2, dans lequel ladite séquence de facteur tissulaire correspond à SEQ ID NO : 6.

17. Procédé selon la revendication 2, dans lequel ladite séquence de facteur tissulaire correspond à SEQ ID NO : 9.

18. Procédé selon la revendication 2, dans lequel ladite séquence de facteur tissulaire correspond à SEQ ID NO : 4.

19. Procédé selon la revendication 2, dans lequel ladite séquence de facteur tissulaire correspond à SEQ ID NO : 7.

20. Procédé selon la revendication 2, dans lequel ladite séquence de facteur tissulaire correspond à SEQ ID NO : 14.

21. Procédé selon la revendication 2, dans lequel ladite séquence de facteur tissulaire correspond à SEQ ID NO : 5.

22. Procédé selon la revendication 2, dans lequel ladite séquence de facteur tissulaire correspond à SEQ ID NO : 8.

23. Procédé selon la revendication 2, dans lequel ladite séquence de facteur tissulaire correspond à SEQ ID NO : 17.

24. Procédé selon la revendication 2, dans lequel ladite séquence de facteur tissulaire correspond à SEQ ID NO : 20.

25. Procédé selon la revendication 2, dans lequel ladite séquence de facteur tissulaire correspond à SEQ ID NO : 23.

26. Procédé selon la revendication 2, dans lequel ladite séquence de facteur tissulaire correspond à SEQ ID NO : 26.

27. Procédé selon la revendication 2, dans lequel ladite séquence de facteur tissulaire correspond à SEQ ID NO : 21.

28. Procédé selon la revendication 2, dans lequel ladite séquence de facteur tissulaire correspond à SEQ ID NO : 24.

29. Procédé selon la revendication 2, dans lequel ladite séquence de facteur tissulaire correspond à SEQ ID NO : 31.

30. Procédé selon la revendication 2, dans lequel ladite séquence de facteur tissulaire correspond à SEQ ID NO : 22.

31. Procédé selon la revendication 2, dans lequel ladite séquence de facteur tissulaire correspond à SEQ ID NO : 25.

32. Procédé selon la revendication 2, dans lequel ladite séquence de facteur tissulaire correspond à SEQ ID NO : 34.

33. Procédé selon la revendication 1, 2 ou 3, dans lequel ledit mélange comprend une composition phospholipidique en conjonction avec ledit échantillon plasmatique.

34. Procédé selon la revendication 33, dans lequel ledit phospholipide comprend une combinaison de phosphatidylcholine et de phosphatidylsérine.

35. Procédé selon la revendication 33, dans lequel ledit phospholipide comprend de la phosphatidylcholine, de la phosphatidylsérine et de la phosphatidyléthanolamine.

36. Procédé selon la revendication 33, dans lequel ladite composition phospholipidique est ajoutée en conjonction avec ledit réactif audit échantillon.

37. Procédé selon la revendication 1, dans lequel lesdits ions calcium sont ajoutés en même temps que ledit réactif audit échantillon.

38. Procédé selon la revendication 1, dans lequel lesdits ions calcium sont ajoutés audit échantillon à la suite dudit réactif.

39. Utilisation d'un réactif à base de facteur tissulaire produit par mutagenèse dans un essai de coagulation pour mesurer directement la concentration en facteur VIII ou facteur IX, dans laquelle ledit réactif à base de facteur tissulaire produit par mutagenèse comprend une séquence d'acides aminés mutante en position 165 et/ou 166 selon la numérotation de SEQ ID NO : 1 et capable de se lier au facteur VIIa et de promouvoir l'activité amidolytique sans être en mesure d'entretenir ou médier l'activation du facteur IX et du facteur X

40. Utilisation selon la revendication 39, dans laquelle ledit réactif à base de facteur tissulaire produit par mutagenèse correspond à SEQ ID NO : 35.

41. Utilisation selon la revendication 40, dans laquelle Xaa dans SEQ ID NO : 35 est choisi dans le groupe consistant en la gltuamine, l'alanine, et l'acide glutamique.

42. Utilisation selon la revendication 39, dans laquelle ledit réactif à base de facteur tissulaire produit par mutagenèse correspond à SEQ ID NO : 36.

43. Utilisation selon la revendication 42, dans laquelle Xaa dans SEQ ID NO : 36 est choisi dans le groupe consistant en la glutamine, l'alanine, et l'acide glutamique.

44. Utilisation selon la revendication 39, dans laquelle le réactif à base de facteur tissulaire produit par mutagenèse

45. Utilisation selon la revendication 44, dans laquelle Xaa dans SEQ ID NO : 37 est choisi dans le groupe consistant en la glutamine, l'alanine, et l'acide glutamique et les combinaisons de ceux-ci.

46. Utilisation selon la revendication 39, dans laquelle ledit réactir à base de facteur tissulaire produit par mutagenèse correspond à SEQ ID NO : 38.

47. Utilisation selon la revendication 46, dans laquelle Xaa dans SEQ ID NO : 38 est choisi dans le groupe consistant en la glutamine, l'alanine, et l'acide glutamique.

48. Utilisation selon la revendication 39, dans laquelle ledit réactif à base de facteur tissulaire produit par mutagenèse correspond à SEQ ID NO : 39.

49. Utilisation selon la revendication 48, dans laquelle Xaa dans SEQ ID NO : 38 est choisi dans le groupe consistant en la glutamine, l'alanine et l'acide glutamique.

50. Utilisation selon la revendication 39, dans laquelle ledit réactif à base de facteur tissulaire produit par mutagenèse correspond à SEQ ID NO : 40.

51. Utilisation selon la revendication 49, dans laquelle Xaa dans SEQ ID NO : 40 est choisi dans le groupe consistant en la glutamine, l'alanine et l'acide glutamique.
